# EUROPEAN PATENT SPECIFICATION

(11) **EP 1 408 949 B1**
(45) Date of publication and mention of the grant of the patent: **19.12.2007**
(21) Application number: 02701110.5
(22) Date of filing: 04.02.2002
(51) Int. Cl.: A61K 31/205, A61P 9/00

(54) **GLYCINE BETAINE AND ITS USE AS ANTI-HEMORRHAGIC AGENT**
ANTIHAEMORRHAGISCHE, BETAIN-ENTHALTENDE ZUSAMMENSEUZUNGEN
GLYCINE-BETAINE ET UTILISATION CORRESPONDANTE

(30) Priority: 05.02.2001 BE 200100085; 31.08.2001 US 945391; 21.12.2001 WO PCT/BE01/00222
(43) Date of publication of application: 21.04.2004
(73) Proprietor: Messadek, Jallal, 4000 Liège (BE)
(72) Inventor: Messadek, Jallal, 4000 Liège (BE)
(86) International application number: PCT/BE2002/000013
(87) International publication number: WO 2002/062322

(56) References cited:
- EP-A- 0 349 902
- WO-A-00/51596
- WO-A-97/06795
- BE-A- 1 012 546
- BE-A- 1 012 712
- US-A- 5 876 780
- PATENT ABSTRACTS OF JAPAN vol. 2000, no. 08, 6 October 2000 (2000-10-06) & JP 2000 143486 A (LION CORP), 23 May 2000 (2000-05-23)
- VINSON, M.C., ET AL.,: "New Drug Approvals of 1996. Part 3" DRUG TOPICS, vol. 141, 1997, pages 7272-81, XP001080754
- DATABASE PHARMPAT [Online] CHEMICAL ABSTRACTS SERVICE, COLUMBUS, OHIO, US; 1995 "naproxen betainate" Database accession no. 19011 XP002202249

## Description

### FIELD OF THE INVENTION

This invention relates to the use of glycine betaine to eliminate physiopathological vascular attacks. The invention relates to the curative and preventive activity of glycine betaine in the pathogenesis of thrombo-embolic and haemostatic diseases of arterial or venous origin.

Glycine betaine exhibits preventive activity while preventing the formation of thrombi and exhibits a curative activity which prevents the proliferation of thrombi while destroying them. The significance of the present invention is based on the fact that the use of glycine betaine does not result in any risk of haemorrhage or allergy in opposition to the molecules and treatments currently used.

### PRIOR ART

Vascular thromboses are a response of the organism which is facing attack on a vessel wall and on the content of cells and plasma thereof. Thrombosis is a localised activation of coagulation with the formation of a thrombus.

The interest to which this pathology has been subjected in recent years has enabled several causative factors to be identified:
- the vessel, the vascular wall and the endothelial cells,
- the role of elements which occur in blood
- the coagulation and fibrinolysis systems, and inhibitors thereof.

Several types of thromboses exist which can occur in arteries, in veins, in the micro-circulation of the organs, in the cavities of the heart and at artificial surfaces in contact with blood. Vascular thromboses are a response to the attack on the vessel wall and on its content of cells and plasma. A thrombosis is an organised mass of blood elements (platelets, red corpuscles and white corpuscles), of fibrin and of other plasma proteins, which are deposited at the surface or which obstruct the free passage of the vascular system.

The mechanisms of thrombosis resemble those of haemostasis, but are pathological due to their abnormal intravascular location.

Thromboses and embolisms are the main reasons for clinical complications associated with cardiovascular diseases and atherosclerosis.

According to Virchow, at least three types of thrombogenetic factors determine the location, the extent and the regression of a thrombosis:
- haemodynamic and rheological factors;
- endothelial lesion;
- activation of the constituents of blood, particularly of platelets, and of coagulation which results in the formation of thrombin.

Thrombo-embolic disease of arterial or venous origin remains one of the main reasons of death in developed countries.

Arterial thrombosis is often due to a rupture of the atherosclerotic plaque, whereas venous thrombosis results from a deficit of a coagulation inhibitor (AT III) or from a deficit of a fibrinolysis activator (S protein and/or C protein) or more frequently from stasis. In fact, both of these result from an interaction between blood and the vascular wall, from the formation of a venous thrombosis and/or from a haemostatic anomaly. Arterial thrombosis is more often secondary to a parietal anomaly and mainly involves blood platelets. It contributes to a wide variety of clinical pictures depending on the arterial layers involved in the interruption of vascularisation. Thrombosis is mainly capable of affecting the cardiac arteries (coronary), and the arteries of the lower, cerebral or digestive organs. Thus arterial disease favours the formation of the thrombus itself which is responsible for the majority of terminal vascular occlusions. Moreover, participation of haemostatic disorders and of the thrombus formed at other vascular lesions is evident: aggravation of the lesions of the vascular wall, ischemia and problems in the micro-circulation.

Three therapeutic strategies can be distinguished for the prevention of accidents associated with thromboses:

Anticoagulants. These constitute the major element in the treatment of a patient exhibiting a thrombo-embolic disorder. Heparin and derivatives thereof are currently used. However, the use of heparins can give rise to two major complications, namely haemorrhage or thrombopenia.

K antivitamins (KAV). Prescribed for long-term treatment, these cannot be used in an emergency and cannot be prescribed simultaneously with other anti-aggregants, since they potentiate the haemorrahgic effect thereof.

Platelet antiaggregants. Prescribed to prevent arterial thrombosis associated with atherosclerosis. The main inhibitors of platelet functioning which are currently prescribed are: aspirin, ticlopidine, dipyridamole, and certain non-steroid anti-inflammatory agents such as flurbiprofen and prostacyclin. These treatments are really effective, but have undesirable effects on patients subject to allergies or haemorrhage.

Despite their efficacy, all these treatments necessitate special precautions in use, such as the administration of antidotes, overdose problems and unwanted side effects. These treatments make it necessary to monitor patients, due in particular to haemorrhage-related problems which can arise during or after medication, as well as possible incompatibility with other drugs. It was therefore of interest to identify a molecule having a high antithrombotic potential without undesirable effects. Most surprisingly, glycine betaine has been identified as possessing a high therapeutic potential for in the treatment of thromboses.

Glycine betaine, or betaine of formula (CH₃)₃N⁺ - (CH₂) - COO⁻, is a molecule known for its osmo-protective properties and for its cosmetic and pharmaceutical uses. Various pharmaceutical uses of betaine are known, particularly the use of betaine for the treatment of homocistinuria, which causes cardiovascular problems (L. & B. Wilken, J. Inher. Metab. Dis. 1997). Thus patients suffering from homocistinuria, which is a genetic anomaly, exhibit premature atherosclerotic and thrombo-embolic disorders (S.H. Mudd et al., The metabolism and molecular bases of inherited disease, 1995), and of cardiovascular diseases (McCully, Atherosclerosis Rev. 11, 1983). Homocistinuria is a hereditary deficiency, the homozygotic form of which is rare. It is estimated that the prevalence of this homozygotic form corresponds to 1 in 200 in the general population.

Homocystinuria is due to elevated levels of homocysteine in the plasma of the affected patient. The administration of betaine enables the concentration of homocysteine in the blood to be reduced.

Publication WO 951 157 50 proposes the use of ingredients comprising betaine in order to prevent vascular disorders in homocistinuric patients.

Publication WO 98 /19690 also relates to patients suffering from an elevated homocysteine level in their blood. The use of betaine amongst other ingredients is intended to reduce the level of homocysteine in the blood, it having been established that homocysteine is a positive factor of risk in the occurrence of cardiovascular diseases, as well as in Alzheimer's disease.

Publication EP 0 347 864 describes the use of betaine together with other ingredients in order to combat the increase in sulfhydryl groups, which are due to cysteine and to homocysteine, in human plasma, and thus to inhibit the formation of atherosclerotic plaques.

This anti-atherosclerotic effect is known and is extensively documented. These publications relate to the effect of betaine on the metabolism of lipids (Zapadnyuk et al. Biol. Med. 1987), and on that of cholesterol (Panteleimonova et al., Farmakol. Toksikol, Moscow 1983).

Publication WO 97 38685 describes the use of betaine and taurine for the treatment of complications resulting from ischemia in some organs. Ischemia is a localised stoppage of the bloodstream and only represents one of the pathologies due to thrombosis.

Publication EP 0 781 554 comprises examples which describe experiments on enucleated hearts, i.e. on hearts which have been extracted and isolated from the vascular system. The use of betaine for its known osmoprotective and antiradical properties enables the inventors to claim a protective action thereof on the cardiac muscle.

Other forms of betaine have been proposed (WO 97 / 06795), but have not hitherto equaled the potency and performance of glycine betaine.

None of these publications discloses the potency of glycine betaine with respect to venous and/or arterial thrombosis, nor its anti-aggregant and anticoagulant potency.

WO 0051596 of applicant, the scope of which is incorporated by reference, discloses the use of betaine for the treatment of thrombosis not induced by homocystinuria. In examples, said application discloses the combination of glycine betaine with a contrast agent.

Said document does not disclose the pharmaceutical combination of a therapeutic agent (an agent for treating a trouble or for preventing a trouble for a patient, especially a mammal) with glycine betaine, nor the advantages of such a combination. Possible advantages of such a combination are the reduction of haemorrahgic side effects and/or potentializing the therapeutic effect of said active agent. It has to be noted that due to reduction of haemorrahgic side effect, it is possible to treat more efficiently the pathology, as the dosage of the therapeutic agent can be increased if required. Furthermore, for drugs, such as antithrombotic drugs, it was observed that the antithrombotic effect of the drug was even potentialized.

Japanese application JP2000143518 describes a skin preparation for external use capable of improving not only a were the rough skin, but also rough skin symptoms associated with atopic dermatosis, drying skin diseases represented by eczema of a housewife or the like, and inflammatory skin diseases, and effective for treating or preventing these rough skin symptoms. This preparation for external use for skin contains a heparin and a betaine, and further an anti-inflammatory agent. JP2000143518 is silent regarding betaine antidote and anti-bleeding properties, nor to its ability for preventing substantially or completely heparin hemorrhagic side effects. In this application no mention is made to oral, parenteral or rectal dosages forms containing a betaine and heparin.

BE-A-1012546 and WO 0051596 of the applicant describe the anti-thrombotic, anti-aggregant and anticoagulant activity of glycine betaine. These applications describe also the use of glycine betaine for the prevention of thrombotic risks derived from the use of contrast agents. Contrast agents are not therapeutically antithrombotic active agents.

WO 97 06795 describes oral or transdermal compositions for the treatment of blood flow disturbances containing butyro-betaine. Butyro-betaine is structurally and physically different of glycine betaine.

Vinson et al in Drugs Topics, volume 141, pages 7272-41, March 17, 1997, in: "New Drugs Approval of 1996 - Part 3" discloses that Cystadane is a commercial pharmaceutical preparation containing betaine as active ingredient for the treatment of homocystinuria. The recommended dosage is of 6 g daily divided in two doses, i.e. one every 12 hours (720 min). Pharmacokinetics studies of betaine by Angela Matthews et al in Br J Clin Pharmacol, 54, 140-146 in: "An indirect response model of homocysteine suppression by betaine: Optimizing the dosage regimen of betaine in homocystinuria" show that betaine is immediately released in the body following oral administration. The plasma concentration of betaine reaches a peak and is rapidly dissolved in the body in a non controlled manner, so the maximum plasma concentration C max (100 % of betaine released) is achieved in 0.9 hour i.e. a T max of 0.9 hour. Thus, Vinson and colleagues do not describe a time controlled or a slow release preparation based on betaine.

Database Pharmpat, Chemical Abstract Service, Colombus, Ohio, US; 1995: 'naproxen betainate', describes a licensed oral composition containing naproxen, a known NSAID agent having haemorrhagic side effects and betaine. This composition is said to have lower gastrointestinal side effects than naproxen alone. Naproxen nor Naproxen/betaine combinations are not prescribed, nor recognised as antithrombotic drugs. In this combination, betaine is used as a buffer to shorten or to lower gastrointestinal mucosa exposition to Naproxen.

The invention relates thus to :
- A pharmaceutical combination comprising a therapeutic effective amount of a therapeutically antithrombotic active agent with at least one haemorrahgic side effect, and a therapeutic effective amount of a compound of formula (CH₃)₃N⁺(CH₂)ₙCOO⁻ with n an integer from 1 to 5, preferably glycine betaine, or a pharmaceutically acceptable salt thereof, and mixtures thereof for preventing or reducing said haemorrahgic side effect and/or for potentializing the therapeutic effect of said active agent;
- Process for the preparation of a pharmaceutical combination of the invention;
- A controlled release pharmaceutical system suitable for delivering in a controlled manner to the bloodstream of a mammalian a betaine or an effective amount of a compound of formula (CH₃)₃N⁺(CH₂)ₙCOO⁻ with n an integer from 1 to 5, preferably glycine betaine or a pharmaceutically acceptable salt thereof, and mixtures thereof; and
- An oral controlled release pharmaceutical system for releasing an effective therapeutically amount of at least a compound selected from the group consisting of betaines or an effective amount of a compound of formula (CH₃)₃N⁺(CH₂)ₙCOO⁻ with n an integer from 1 to 5, pharmaceutically acceptable salts thereof, and mixtures thereof, for treating or preventing blood flow disturbances.

### BRIEF DESCRIPTION OF THE INVENTION

Glycine betaine, as well as betaine compounds of the general formula (CH₃)₃N⁺- (CH₂)ₙ- COO⁻, with n varying from 1 to 5 (preferably equal to I) in the context of the present invention can be used for various clinical applications, such as: coronary thromboses and venous thromboses thromboses and re occlusion of the vascular system following a thrombolysis or an angioplasty
- infarct, angina pectoris, aneurysm, pulmonary embolism, phlebitis
- cerebral embolism
- post-traumatic shock, whether or not of surgical origin
- prevention of accidents of microcirculation in the following cases: haemophilia, chemotherapy, ageing, oral contraception using oestrogens, obesity, tobacco addiction, prosthesis, diabetes.

Platelet aggregation is an essential event in the formation of blood clot and thrombus. In normal conditions, following a vascular lesion, blood clots prevent blood losses by closing the opening. However, in some pathological instances, the formation of a blood clot can reduce partly or completely the blood circulation, with the consequence of a cellular necrosis.

For example, the platelet aggregation and thus the thrombosis at the level of the artherosclerosis plaques is an important factor for the genesis of conditions such as angina pectoris, myocardus infarct, vessel occlusion following a thrombolysis or an angioplasty. Patients suffering a heart attack are treated with thrombolytic agents such as plasmin activators and the streptokinases which dissolve the fibrin from the clots. A major complication of this therapy is the reocclusion of vessels due to platelet aggregation, which can lead to irreversible damages to the heart, the brain or other organs.

Thrombosis starts with the adhesion of platelets at the vascular lesion sites.The platelet adhesion is initiated by the receptor located at the surface of the platelets which bind to proteins of the extracellular cellular matrix of the exposed endothelium, such as fibrinogen, fibronectin, Von Willebrand factor, as well as other adhesive proteins such as vibronectin, collagen and laminin. Therefor, the activation of platelets is a reply to agonists such as epinephrine, ADP, collagen, the arachidonic acid or the thrombin. This activation leads to the activation of the glycoprotein Ib receptor (GP Ib) and/or of the glycoprotein IIb IIIa receptor (GP IIb IIIa) at the surface of the platelets. This receptor(s) (GP Ib and/or GP IIb IIIa) is/are then available for its/their binding to fibrinogen and the platelet aggregation. The adhesion of the receptor (GP IIb IIIa) to other adhesive proteins such as the Von Willebrand factor also leads the attachment of platelets between them and their aggregation. The adhesion of molecules such as fibrinogen or the Von Willebrand factor to the receptor (GP IIb IIIa) leading the platelet aggregation is an essential step in the formation of the thrombus. The receptor (GP IIb IIIa) is thus a privileged target for the new therapy treating thrombosis and thromboembolitic pathologies. Furthermore, the use of antagonists of the glycoprotein IIb IIIa receptor inhibits the platelet aggregation, while respecting the other hemostasis mechanisms, is highly desirable in the new therapies bound to thrombosis. Several molecules having this antagonist property are marketed with usage restrictions due to immunoreactivity problems, toxicity, allergy or hypersensibility reactions for some patients. A subject matter of the present invention is to propose a molecule, especially a well-known and used molecule of vegetal origin, having this antagonist activity for the glycoprotein IIb IIIa receptor, while not having toxic characteristics.

It is also known that the activation of the vitronectin receptor improves the cell migration and provides regulating signals of the cell proliferation and cell differentiation, and activates the effects of insulin (Ruoslahti, Kidney Int., 1997, 51, 1413-1417). The regulation of the vitronectin receptor is associated with pathological conditions, such as vascular restinosis (Clemetson and Clemetson, Cell.Mol. Life Sci., 1998,54,502-513), bone excess resorbtion (Rodan and Rodan, J. Endocrinol., 1997, 154 Suppl, S47-56), and the angiogenesis process during the malignant melanomas (Cheresh, Cancer Metastasis Rev., 1991, 10,3-10).

Surprisingly, it has now been found that betaines of formula (CH₃)₃N⁺- (CH₂)ₙ-COO⁻, with n an integer from 1 to 5, and their pharmaceutically acceptable salts, have an antagonist activity for one or more glycoprotein(s) receptors, such as the glycoprotein Ib receptor and the glycoprotein IIb IIIa receptor, by inhibiting the platelet aggregation induced by various agonists. This antagonist activity is not restricted to the glycoprotein site IIb IIIa but to all glycoprotein sites implicated in the cell adhesion of various origins, there between.

Platelets are activated by some agonists, whereby their forms, as well as their secretions of their granules can be modified, and whereby the aggregation thereof can be induced and the formation of clots and thrombi can be produced.

Several endogenous agonists, such as ADP (adenosine-5-diphosphate), serotinine, arachidonic acid, epinephrine, adrenaline, thrombin, collagen, ristocetine are known.

Recently a mechanism of action of these agonists has been identified, namely the activation of the glycoproteic site GP IIb IIIa which causes the adhesion of the circulating fibrinogen (Thromb. Res. 1993, 72, 231-245) and therefore the consolidation of platelets groups and the formation of clot. (Drug of the future, 1994, 19 (2), 135-159)

The actually used platelet aggregation inhibitors are acting only on a single agonist. For example, aspirin is active against the arachidonic acid, ticlopidin is active against ADP, hirudin is active against thrombin. The betaines of the general formula of the invention disclosed here before are actives against various agonists, as well as on fibrinogen, fibronectin, Von Willebrand factor and other adhesive proteins such as vitronectin, collagen, laminin. This is a major improvement for their efficiency, while preserving the hemostasis mechanism so as to avoid haemorrahgic or bleeding events. Due to their activity by oral administration, said compounds are excellent candidates for pathologies with adhesion of cells between them.

In view of its very low toxicity and its efficiency, the best results have been obtained with glycine betaine (compound of the general formula with n = 1).
None of the publications to which reference is made in the present specification teach the antagonist activity of the betaine with respect to glycoprotein IIb IIIa receptor, nor its activity with respect to adhesive proteins. This antagonist activity is not only limited to the site of glycoprotein IIb IIIa, but also to all the other glycoproteic sites acting in the adhesion of cells of various origins there between.

In the present specification, pharmaceutically acceptable salts are salts of betaine which can be administered, such as salts of betaine with hydrochloric acid, sulfuric acid, sulfonic acid, organic acids such as acetic acid, citric acid, tartaric acid, formic acid, etc., as well as the monohydrate radical.

Betaines, preferably glycine betaine, is advantageously administered orally, parenterally, sub cutaneously, by suppositories, tablets, capsules, syrup, etc.

Administered doses can vary from 0.001g to 10 g per kg live body, for example from 0.005 g to 5 g, in particular from 0.01 g to 3 g per kg life body.

Examples of administration forms are : tablets, capsules, patches, injectable forms, releasing forms, sublingual administration form, powder (for example for inhalation therapy, buccal inhalation), syrup, solution (nebulization, for example for inhalation therapy, buccal inhalation). As preferred administration forms, subcutaneous injectable dosage form, patches (to be applied on the skin) and entero soluble oral dosage form, such as gastro insoluble tablets or capsules, etc. provided with an entero soluble coating or matrix or system.

As the pH of an aqueous glycine betaine solution is comprised between about 6 and about 7, an injectable solution (preferably for a subcutaneous injection) can be prepared by mixing solid glycine betaine with water (sterilized and possibly demineralized). The glycine betaine can be in the form of a powder (lyophilized powder) placed in a vial, water is then added to said vial for the preparation of the solution to be injected. If necessary, some acid (such as hydrochloric) can be added to the solution or to the water to be mixed with the powder.

The injectable dosage form can be a pressurized dosage form, such as an air pressurized dosage form. Subcutaneous injectable forms of glycine betaine, such as intravenous injectable forms, are preferred. Glycine betaine injectable forms are for example aqueous solution containing 0.1 to 50% by weight glycine betaine, advantageously from 0.5 to 30%, preferably from 10 to 20%. The injectable form has a pH for example comprised between 5 and 8.5, advantageously from 6 to 7.5, preferably from 6 to 6.5. When the injectable form is prepared by mixing glycine betaine (as a solid form or as a powder form), the pH of the solution is about 6-6.5.

When the glycine betaine is administered by injection, the glycine betaine can be present in a solution of a flexible bag (baxter), for example a flexible bag (baxter) for intravenous administration of a saline solution, or a physiological solution, or a blood transfusion baxter.

The invention relate thus also to a bag (flexible bag or baxter) for subcutaneous administration (preferably intravenous administration) containing a solution suitable for subcutaneous administration. As more specific example, the bag or baxter contains blood or a blood derivative or a blood portion and glycine betaine for subcutaneous administration.

Another subject matter of the invention is a pharmaceutical composition (such as a tablet) containing insulin and betaine, a pharmaceutical composition (such as a tablet) containing an antibiotic and betaine, a pharmaceutical composition (such as a tablet) containing an anti cancerous agent and betaine, a pharmaceutical composition (such as a tablet) containing aspirin and betaine, etc.

A subject matter of the invention is thus a pharmaceutical combination as claimed in claim 1 comprising an effective amount of a therapeutical antithrombotic active agent causing at least a haemorrahgic side effect, and an effective amount of a compound of formula (CH₃)₃N⁺-(CH₂)ₙ-COO⁻, with n an integer from 1 to 5, preferably equal to 1, for preventing at least 50%, advantageously at least 75%, preferably at least 90%, most preferably substantially completely said side effect and/or for reducing the seriousness of said side effect, advantageously of a factor of at least 50%, preferably of at least 75%, most preferably of at least 90%, especially substantially completely.

Betaine is preferably used as anti haemorrahgic agent in said combination.

Betaine is preferably used as an antitode to an haemorrahgic agent in said combination.
The pharmaceutical combination can be in the form of a kit, so as to prepare the combination before administration or during the administration.
Side effect is defined as being events observed more than 2% for the patients in treatment with the active agent. By combining said active agent with betaine, it is possible to reduce drastically said events, for example to less than 2%, as well as the importance or gravity of said events.
The active agent with possible side effect is selected among the group consisting of, anti inflammatory agents, anti aggregation agents, anti coagulation agents, anti thrombotic agents, thrombolytic agents, Tpa agents, anti cholesterol agents, anti vitamin K and mixtures thereof. Specific examples of such agents are glycoaminoglycans, heparins (such unfractioned heparin, standard heparin, low molecular heparins, heparinoid, and mixtures thereof), heparin-like molecules (such as heparinoid, danaparoid, orgaran, fragmin, dalteparin, enoxaparine, lovenox, ardeparin, normiflo and mixtures thereof), thrombin inhibitor (such as argatroban, novastan, and mixtures thereof), aspirin, anti aggregation agents, anti coagulation agents, antiplatelet agents (such as dextrans, dipryridamole, sulfinpyrazone, ticlodipine, abcximab, tirofiban, mixtures thereof), anti thrombotic agents, thrombolytic agents (such as human recombinant activated protein, tissue plasminogen activator, urokinase, streptokinase, anistreplase/APSAC, and mixtures thereof), anti cholesterol agents, anti vitamin K, Tpa agents (tissue plasminogen activator), glycoaminoglycans, heparinoid agents, hirudins, warfarins, coumadin, coumarin, agents of the statin family, ticlopidine, statin agents, cerivastatine, simvastatin, lovastatin.

The invention relates also to the use of a compound of formula (CH₃)₃N⁺(CH₂)ₙCOO⁻ with n an integer from 1 to 5, preferably glycine betaine or a pharmaceutically acceptable salt thereof, and mixtures thereof, as active antidote agent for the preparation of an antidote composition, for example for combating haemorrahgic troubles, such as haemorrahgic troubles caused by one or more agents selected from the group consisting of:
contrast agents, anti inflammatory agents, anti aggregation agents, anti coagulation agents, anti thrombotic agents, thrombolytic agents, Tpa agents, anti cholesterol agents, anti vitamin K and mixtures thereof. Specific examples of such agents are glycoaminoglycans, heparins (such unfractioned heparin, standard heparin, low molecular heparin, heparinoid, and mixtures thereof), heparin-like molecules (such as heparinoid, danaparoid, orgaran, fragmin, dalteparin, enoxaparine, lovenox, ardeparin, normiflo and mixtures thereof), thrombin inhibitor (such as argatroban, novastan, and mixtures thereof), aspirin, anti inflammatory agents (such as non steroid anti inflammatory agents), anti aggregation agents, anti coagulation agents, antiplatelet agents (such as dextrans, dipryridamole, sulfinpyrazone, ticlodipine, abcximab, tirofiban, mixtures thereof), anti thrombotic agents, thrombolytic agents (such as human recombinant activated protein, tissue plasminogen activator, urokinase, streptokinase, anistreplase/APSAC, and mixtures thereof), anti cholesterol agents, anti vitamin K, Tpa agents (tissue plasminogen activator), glycoaminoglycans, heparinoid agents, hirudins, anti vitamin K, warfarins, coumadin, coumarin, agents of the statin family, ticlopidine, statin agents, cerivastatine, simvastatin, lovastatin, agents of the statin family, cerivastatine (Baycol), simvastatin, lovastatin, etc.

Antithrombotic and/or non haemorrahgic formulations in the scope of the present invention can be a combination of therapeutically effective amount of compound(s) of formula (CH₃)₃N⁺(CH₂)ₙCOO⁻ with n an integer from 1 to 5, pharmaceutically acceptable salts thereof, and mixtures thereof, and therapeutically effective amount of one or more compound(s), pharmaceutically acceptable salts thereof, esters thereof, precursors thereof and mixtures thereof, selected from the group consisting of:
anti aggregants as
abciximab, acetylsalicylate basic aluminium, acetylsalicylate carbonate sodium, acetylsalicylate lysine, acetylsalicylic acid, aloxiprine, anagreli chlorydrate, bencyclane furamate, carbasalate calcium, clopidogrel sulfate, epoprostenol sodium, epifibati, hydroxychloroquine sulfate, iloprost, nicergoline, nifepidine, pyricarbate, sulfinpyrazone, ticlopidine chlorhydrate, tirofiban chlorhydrate, verapamil chlorhydrate, and compounds structurally similar to one of the preceding anti aggregant compounds,
and/ or anticoagulants as
acenocoumarol, anisindione, biscoumacetate ethyl, bromindione, coumetarol, dalteparine sodium, sirudine, xtran sulfate, enoxaparine sodium, fluindione, heparinate magnesium, heparin calcium, heparine sodium, lepirudine nadroparine calcium, oxazidione, pentosane polyester sulfuric, phenindione, phenprocoumone, reviparine sodium, tinzaparine sodium, tioclomarol, warfarine sodium, and compounds structurally similar to one of the preceding anti coagulant compounds,
and/ or fibrinolytics as
altepase, anistreplase, atorvastatine calcium, bromelaines, ciprofibrate, defibrotide, fluvastatine sodium, glicazide, lovastatine, lys-plasminogene, phenformine, pravastatine sodium, reteplase, simvastatine, streptokinase, urokinase, and compounds structurally similar to one of the preceding fibrinolytic compounds.

These antithrombotic and/or non haemorrahgic formulations can be preparations for oral, rectal, parenteral, transdermal, extracorporal, intracorporal administration. For example, for said combinations, the weight ratio between [compound(s) of formula (CH₃)₃N⁺(CH₂)ₙCOO⁻ with n an integer from 1 to 5, pharmaceutically acceptable salts thereof, esters thereof, precursors thereof and mixtures thereof] / [anti aggregants agents and /or anticoagulant agents and/or fibrinolytic agents and/or mixture there of] is comprise between 50/1 and 1/50, advantageously between 25/1 and 1/2, preferably between 10/1 and 1/1. In one embodiment of the present invention, betaine due to its antithrombotic properties may be used to ameliorate the antithrombotic effect of the cited above anti aggregants and/or anticoagulant and/or fibrinolytic agents.
The combined pharmaceutical form of antidote can be a combination of anti coagulant antagonists (such as protamine, vitamine K1, mixtures thereof), and/or thrombolytic agent antagonists (amiocaproic acid, tranexamic acid and mixtures thereof) and a compound of formula (CH₃)₃N⁺(CH₂)ₙCOO⁻ with n an integer from 1 to 5, pharmaceutically acceptable salts thereof, esters thereof, precursors thereof and mixtures thereof.
These combined forms of antidote can be preparations for oral, rectal, parenteral, transdermal, extracorporal, intracorporal administration.

The antithrombotic and/or non haemorrahgic formulations as described above can be combined to the combined pharmaceutical form of antidote as described above.

In one embodiment of the present invention, a compound of formula (CH₃)₃N⁺(CH₂)ₙCOO⁻ with n an integer from 1 to 5, pharmaceutically acceptable salts thereof, and mixtures thereof due to it's anti haemorrahgic properties may be used to treat haemophilia.
In one embodiment of the present invention, a compound of formula (CH₃)₃N⁺(CH₂)ₙCOO⁻ with n an integer from 1 to 5 due to it's anti haemorrahgic properties may be used in combination to ameliorate the anti haemophilia drugs potency.

The combined pharmaceutical form can be a form in which the active agent and the betaine are administered simultaneously or successively, using the same administration way or different administration ways. As specific examples, when using different administration ways, the betaine is administered in the form of a patch or by subcutaneous injection, while the other active agent is administered by oral way or by injection (subcutaneous, venous). When the combined pharmaceutical form is administered using the same administration way, the dosage form is advantageously an injectable form (such as a venous injectable form), but is preferably a oral dosage form, most preferably a solid or semi-solid dosage form. When using a dosage form, the active agent is advantageously in the form of pellets or micropellets or particles which are coated with a betaine containing layer. The coated particles or pellets can be further be coated with an enterosoluble coating which is gastric insoluble or placed in a matrix or capsule which is enterosoluble and gastric insoluble. Preferably, at least the glycine betaine is in a form suitable for subcutaneous injection (preferably intra venous injection) or in a form adapted for the preparation of a form suitable for subcutaneous injection (preferably intravenous injection)

The invention further relates to a process for the preparation of a composition of the invention of treatment of a patient in need or for for reducing or preventing, the hemorrhagic side effect caused by administering to said patient a therapeutic effective amount of an therapeutic active agent with at least one hemorrhagic side effect, by using a therapeutic effective amount of a compound of formula (CH₃)₃N⁺-(CH₂)ₙ-COO⁻, with a n an integer from 1 to 5, preferably equal to 1, for preventing at least 50%, advantageously at least 75%, preferably at least 90%, most preferably substantially completely said troubles and/or for reducing the seriousness of said side effect, advantageously of a factor of at least 50%, preferably of at least 75%, most preferably of at least 90%, especially substantially completely. Glycine betaine is preferably subcutaneous injected (most preferably intravenous injection)
The present invention relates to a controlled release preparation system or device of betaine and, to processes for its preparation and to its medical use. In particular, the invention relates to a controlled release preparation comprising betaine, preferably glycine betaine or a pharmaceutically acceptable salt thereof.

Betaine is a compound of formula (CH₃)₃ N⁺ (CH₂)ₙ COO⁻ with n being an integer of 1 to 5. Conventional preparations in the form of syrup, or powder have been commercially available for many years for use in the treatment of homocystinuria. Such preparations, however, do not provide a controlled release of the betaines. Moreover, despite 's long-standing use, controUed release preparations for oral, rectal, parenteral, transdermal, extracorporal, intracorporal administration containing as active ingredient a betaine have not even previously been described, nor suggested in the literature.

It is an object of the present invention to provide an oral, rectal, parenteral, transdermal, extracorporal, intracorporal controlled release of a betaine, preferably glycine betaine preparation suitable for at least twelve-hourly (e.g. up to twenty-four hourly or even more, such as for a week, two weeks, one month, three months) administration for the treatment of a mammalian.

The present invention therefore provides a controlled release preparation and/or device comprising betaine, preferably glycine betaine or a pharmaceutically acceptable salt thereof or ester thereof for body (oral, rectal, parenteral, transdermal, extracorporal, intracorporal, etc.) administration.

Suitable pharmaceutically acceptable salts of betaine, preferably glycine betaine for use according to the present invention are those conventionally known in the art such as pharmaceutically acceptable acid addition salts. The anhydrous salt is particularly preferred.
The invention relates to a controlled release pharmaceutical system suitable for delivering in a controlled manner to the bloodstream of a mammalian a betaine or an effective amount of a compound of formula (CH₃)₃N⁺(CH₂)ₙCOO⁻ with n an integer from 1 to 5, preferably glycine betaine or a pharmaceutically acceptable salt thereof, and mixtures thereof, and/or to
a controlled release pharmaceutical system suitable for delivering in a controlled manner to the bloodstream of a mammalian as active ingredient at least a compound selected from the group consisting of betaines or an effective amount of a compound of formula (CH₃)₃N⁺(CH₂)ₙCOO⁻ with n an integer from 1 to 5, pharmaceutically acceptable salts thereof, and mixtures thereof.
The system of the invention is an oral controlled release preparation or device and/or a transdermal controlled release preparation and/or a transdermal controlled release device, and/or
a parenteral controlled release preparation and/or an extracorporal controlled release device and/or an intracorporal controlled release device or preparation and/or a rectal controlled release preparation and/or a rectal controlled release device and/or a mucous controlled release preparation or device and/or a pulmonary controlled release preparation or device and/or an ocular controlled release preparation or device.
According to an embodiment, the system combines at least two systems selected from the group consisting of: oral controlled release preparations, oral controlled release device, transdermal controlled release preparations, transdermal controlled release devices, parenteral controlled release preparations, parenteral controlled release devices, extracorporal controlled release preparations, extracorporal controlled release devices, intracorporal controlled release preparations, intracorporal controlled release devices, rectal controlled release preparations, rectal controlled release device, a mucous controlled release preparations, mucous controlled release devices, pulmonary controlled release preparations, pulmonary controlled release devices, ocular controlled release preparations and ocular controlled release devices.

In the system of the invention the active ingredient is preferably glycine betaine.
The invention relates also to a controlled release pharmaceutical system for releasing an effective therapeutically amount of at least a compound selected from the group consisting of betaines or an effective amount of a compound of formula (CH₃)₃N⁺(CH₂)ₙCOO⁻ with n an integer from 1 to 5. pharmaceutically acceptable salts thereof, and mixtures thereof, for treating or preventing blood flow disturbances, and/or for treating or preventing thrombosis and/or thromboembolic disorders.

A controlled release system of the invention advantageously controls at least for 120 minutes, advantageously at least for 180 minutes, preferably at least for 240 minutes, the release of glycine betaine or an effective amount of said compound, or pharmaceutically acceptable salts thereof, or and mixtures thereof.
Most preferably the system controls at least for 180 minutes, advantageously at least for 240 minutes, preferably at least for 360 minutes, most preferably at least for 1440 minutes (for example for 1, week, for two weeks, for one month), the release of at least a glycine betaine or an effective amount of a glycine betaine, pharmaceutically acceptable salts thereof, and mixtures thereof.

According to a possible embodiment, the system and/or the device of the invention comprises one or more electronic device or chips controlling one or more releasing system or device, such as a micro pump(s), a syringe, a balloon, etc. The invention further relates to:
- Process for the preparation of a pharmaceutical composition for treating or preventing a trouble caused by administering to a patient an effective amount of a therapeutic active agent with at least one haemorrhagic side effect, in which an effective amount of a compound of formula (CH₃)₃N⁺(CH₂)ₙCOO⁻ with n an integer from 1 to 5, or a pharmaceutically acceptable salt thereof, or mixtures thereof is used as therapeutic active agent for preventing or reducing said side effect and/or for potentialising the therapeutic effect of said therapeutically active agent, and
- Process for the preparation of a pharmaceutical composition for treating or for preventing thrombosis troubles in a patient, by administering to said patient a therapeutic effective amount of an anti thrombotic active agent with at least one haemorrhagic side effect, in which a therapeutic effective amount of glycine betaine is used in active agent for preventing or reducing said haemorrhagic side effect.

An example of oral controlled release preparation according to the present invention is one that achieves slow release of a drug over an extended period of time, thereby extending the duration of drug action over that achieved by conventional delivery. Preferably such a preparation maintains a drug concentration in the blood within the therapeutic range for 12 hours or more, most preferably for 24 hours or more.

The present inventor has found that in order to allow for controlled release betaine, preferably glycine betaine over at least a twelve hour period following oral administration, the in vitro release rate preferably corresponds to the following % rate of betaine, preferably glycine betaine released:

**TABLE 1**

| TIME(H) | % RELEASED |
|---|---|
| 1 | 0-50 |
| 2 | 0-75 |
| 4 | 3-95 |
| 8 | 10-100 |
| 12 | 20-100 |
| 16 | 30-100 |
| 24 | 50-100 |
| 36 | >80 |

Another preferred preparation especially suited for twice-a-day dosing has an in vitro release rate corresponding to the following % rate of betaine, preferably glycine betaine released:

**TABLE 2**

| TIME (H) | % RELEASED |
|---|---|
| 1 | 20-50 |
| 2 | 40-75 |
| 4 | 60-95 |
| 8 | 80-100 |
| 12 | 90-100 |

Yet another preferred preparation particularly suited for once-a-day dosing has an in-vitro release rate corresponding to the following % rate of betaine, preferably glycine betaine released:

**TABLE 3**

| TIME (H) | % RELEASED |
|---|---|
| 1 | 0-50 |
| 2 | 0-75 |
| 4 | 10-95 |
| 8 | 35-100 |
| 12 | 55-100 |
| 16 | 70-100 |
| 24 | >90 |

A still farther preferred preparation in accordance with the invention also particularly suited for once-a-day dosing has an in vitro release rate corresponding to the following % rate of betaine, preferably glycine betaine released.

**TABLE 4**

| TIME(H) | % RELEASED |
|---|---|
| 1 | 0-30 |
| 2 | 0-40 |
| 4 | 3-55 |
| 8 | 10-65 |
| 12 | 20-75 |
| 16 | 30-88 |
| 24 | 50-100 |
| 36 | >80 |

More preferably a preparation for once-a-day dosing has an in vitro release rate substantially as follows;

| TIME (H) | % BETAINE, PREFERABLY GLYCINE BETAINE RELEASED |
|---|---|
| 1 | 15-25 |
| 2 | 25-35 |
| 4 | 30-45 |
| 8 | 40-60 |
| 12 | 55-70 |
| 16 | 60-75 |

Another preferred dissolution rate in vitro upon release of the controlled release preparation for administration twice daily according to the invention, is between 5 and 50% (by weight) betaine, preferably glycine betaine released after 1 hour, between 10 and 75% (by weight) betaine, preferably glycine betaine released after 2 hours, between 20 and 95% (by weight) betaine, preferably glycine betaine released after 4 hours, between 40 and 100% (by weight) betaine, preferably glycine betaine released after 8 hours, more than 50% (by weight) betaine, preferably glycine betaine released after 12 hours, more than 70% (by weight) released after 18 hours and more than 80% (by weight) betaine, preferably glycine betaine released after 24 hours.

Furthermore, it is preferred in the case of a controlled release preparation for administration twice daily that after 8 hours following oral administration between 70 and 95% (by weight) betaine, preferably glycine betaine is absorbed in vivo, between 77 and 97% (by weight) betaine, preferably glycine betaine is absorbed after 10 hours and between 80 and 100% (by weight) betaine, preferably glycine betaine is absorbed after 12 hours.

A formulation in accordance with the invention suitable for twice-a-day dosing may have a tmax of 1.5 to 8 hours, preferably 2 to 7 hours, and a W.sub.50 value in the range 7 to 16 hours.

A formulation in accordance with the invention suitable for once-a-day dosing may have a tmax in the range of 3 to 6 hours, preferably 4 to 5 hours and a W.sub.50 value in the range of 10 to 36 hours.

The W.sub.50 parameter defines the width of the plasma profile at 50% Cmax, i.e. the duration over which the plasma concentrations are equal to or greater than 50% of the peak concentration. The parameter is determined by linear interpolation of the observed data and represents the difference in time between the first (or only) upslope crossing and the last (or only) downslope crossing in the plasma profile.

The in vitro release rates mentioned herein can be are, except where otherwise specified, those obtained by measurement using the Ph. Eur. Paddle Method. at 100 rpm in 900 ml 0.1N hydrochloric acid at 37.degree. C. and using any suitable method for detecting the betaine or glycine betaine (such as HPLC, UV detection, etc.,).

The in vivo absorption rate is determined from measurement of plasma concentration against time using the deconvolution technique. A conventional release betaine, preferably glycine betaine drop preparation was used as the weighting-function and the elimination half life of betaine, preferably glycine betaine was taken as 7.8 hours.

The controlled release preparation according to the invention preferably contains an effective amount of betaine, preferably glycine betaine or a pharmaceutically acceptable salt or esters thereof, precursors thereof, precursors thereof, conveniently in the range of from 50 to 8000 mg, especially 100, 200, 300, 400 to 600, 800 to 1000, 1500 to 5000 mg (calculated as betaine, preferably glycine betaine anhydrous) per dosage unit.

The controlled release preparation according to the invention may be presented, for example, as granules, spheroids, pellets, multiparticulates, capsules, tablets, sachets, controlled release suspensions, or in any other suitable dosage form incorporating such granules, spheroids, pellets or multiparticulates.

The active ingredient in the preparation according to the invention may suitably be incorporated in a matrix. This may be any matrix that affords controlled release betaine, preferably glycine betaine over at least a twelve hour period and preferably that affords in-vitro dissolution rates and in vivo absorption rates of betaine, preferably glycine betaine within the ranges specified above. Preferably the matrix is a controlled release matrix. Alternatively, normal release matrices having a coating which provides for controlled release of the active ingredient may be used.

Suitable materials for inclusion in a controlled release matrix include
(a) Hydrophilic or hydrophobic polymers, such as gums, cellulose ethers, acrylic resins and protein derived materials. Of these polymers, the cellulose ethers, especially alkylcelluloses are preferred. The preparation may conveniently contain between 1% and 80% (by weight) of one or more hydrophilic or hydrophobic polymers.
(b) Digestible, long chain (C₈ -C₅₀, especially C₁₂ -C₄₀), substituted or unsubstituted hydrocarbons, such as fatty acids, fatty alcohols, glyceryl esters of fatty acids, mineral and vegetable oils and waxes. Hydrocarbons having a melting point of between 25.degree. and 90.degree. C. are preferred. Of these long chain hydrocarbon materials, fatty (aliphatic) alcohols are preferred. The preparation may conveniently contain up to 60% (by weight) of at least one digestible, long chain hydrocarbon.
(c) Polyalkylene glycols. The preparation may suitably contain up to 60% (by weight) of one or more polyalkylene glycols.

One particularly suitable controlled release matrix comprises one or more alkylcelluloses and one or more C₁₂ -C₃₆ aliphatic alcohols. The alkylcellulose is preferably C₁ -C₆ alkyl cellulose, especially ethyl cellulose. The controlled release preparation according to the invention preferably contains from I to 20% (by weight), especially from 2 to 15% (by weight) of one or more alkylcelluloses.

The aliphatic alcohol may conveniently be lauryl alcohol, myristyl alcohol or stearyl alcohol but is preferably cetyl alcohol or more preferably cetostearyl alcohol. The controlled release preparation suitably contains from 5 to 30% (by weight) of aliphatic alcohol, especially from 10 to 25% (by weight) of aliphatic alcohol.

Optionally the controlled release matrix may also contain other pharmaceutically acceptable ingredients which are conventional in the pharmaceutical art such as diluents, lubricants, binders, granulating aids, colorants, flavorants, surfactants, pH adjusters, anti-adherents and gildants, e.g. dibutyl sebacate, ammonium hydroxide, oleic acid and colloidal silica.

The controlled release preparation according to the invention may conveniently be film coated using any film coating material conventional in the pharmaceutical art. Preferably an aqueous film coating is used.

Alternatively, the controlled release preparation according to the invention may comprise a normal release matrix having a controlled release coating. Preferably the preparation comprises film coated spheroids containing the active ingredient and a spheronising agent.

The spheronising agent may be any suitable pharmaceutically acceptable material which may be spheronised together with the active ingredient to form spheroids. A preferred spheronising agent is microcrystalline cellulose. The microcrystalline cellulose used may suitably be, for example, Avicel PH 101 or Avicel PH 102 (Trade Marks, FMC Corporation).

Optionally the spheroids may contain other pharmaceutically acceptable ingredients conventional in the pharmaceutical art such as binders, bulking agents and colorants. Suitable binders include water soluble polymers, water soluble hydroxyalkyl celluloses such as hydroxypropylcellulose or water insoluble polymers (which may also contribute controlled release properties) such as acrylic polymers or copolymers for example ethylcellulose. Suitable bulking agents include lactose.

The spheroids are coated with a material which permits release of the active ingredient at a controlled rate in an aqueous medium. Suitable controlled release coating materials include water insoluble waxes and polymers such as polymethacrylates (for example Eudragit polymers, Trade Mark) or water insoluble celluloses, particularly ethylcellulose. Optionally, water soluble polymers such as polyvinylpyrrolidone or water soluble celluloses such as hydroxypropylmethylcellulose or hydroxypropylcellulose may be included. Optionally other water soluble agents such as polysorbate 80 may be added.

Alternatively the drug may be coated onto inert non-pareil beads and the drug loaded beads coated with a material which permits control of the release of the active ingredient into the aqueous medium.

In a further aspect the present invention provides a process for preparing a controlled release preparation according to the present invention comprising incorporating betaine, preferably glycine betaine or a pharmaceutically acceptable salt thereof in a controlled release matrix:
(a) granulating a mixture comprising betaine, preferably glycine betaine or a pharmaceutically acceptable salt thereof and one or more alkylcelluloses,
(b) mixing the alkylcellulose containing granules with one or more C₁₂₋₃₆ aliphatic alcohols; and optionally
(c) shaping and compressing the granules, and film coating, if desired; or
(d) granulating a mixture comprising betaine, preferably glycine betaine or a pharmaceutically acceptable salt thereof, lactose and one or more alkylcelluloses with one or more C₁₂₋₃₆ aliphatic alcohol; and, optionally,
(e) shaping and compressing the granules, and film coating, if desired.

The controlled release preparation according to the invention may also be prepared in the form of film coated spheroids by
granulating the mixture comprising betaine, preferably glycine betaine or a pharmaceutically acceptable salt thereof and a spheronising agent;
extruding the granulated mixture to give an extrudate;
spheronising the extrudate until spheroids are formed; and
coating the spheroids with a film coat.

One preferred form of unit dose form in accordance with the invention comprises a capsule filled with controlled release particles essentially comprising the active ingredient, a hydrophobic fusible carrier or diluent and optionally a hydrophilic release modifier. In particular, the controlled release particles are preferably prepared by a process which comprises forming a mixture of dry active ingredient and fusible release control materials followed by mechanically working the mixture in a high speed mixer with an energy input sufficient to melt or soften the fusible material whereby it forms particles with the active ingredient. The resultant particles, after cooling, are suitably sieved to give particles having a size range from 0.1 to 3.0 min, preferably 0.25 to 2.0 mm. An example according to the invention is described below which is suitable for the commercial production of dosage units.

When using such a processing technique it has been found that, in order most readily to achieve the desired release characteristics (both in vivo and in vitro as discussed
above) the composition to be processed should comprises two essential ingredients namely:
betaine, preferably glycine betaine or salt thereof; and
hydrophobic fusible carrier or diluent; optionally together with
a release control component comprising a water-soluble fusible material
or a particulate soluble or insoluble organic or inorganic material.

The inventor found that the total amount of betaine, preferably glycine betaine or pharmaceutically acceptable salt thereof in the composition may vary within wide limits, for example from 10 to 90% by weight thereof.

The hydrophobic fusible component (b) should be a hydrophobic material such as a natural or synthetic wax or oil, for example hydrogenated vegetable oil, hydrogenated castor oil, microcrystalline wax, Beeswax, Carnauba wax or glyceryl monostearate, and suitably has a melting point of from 35°C. to 140°C., preferably 45°C to 110°C.

The release modifying component (c), when a water soluble fusible material, is conveniently a polyethylene glycol and, when a particulate material, is conveniently a pharmaceutically acceptable material such as dicalcium phosphate or lactose.

Another preferred process for the manufacture of a formulation in accordance with the invention comprises
(a) mechanically working in a high-speed mixer, a mixture of betaine, preferably glycine betaine or a pharmaceutically acceptable salt in particulate form and a particulate, hydrophobic fusible carrier or diluent having a melting point from 35°C to 140°C. and optionally a release control component comprising a water soluble fusible material, or a particulate soluble or insoluble organic or inorganic material at a speed and energy input which allows the carrier or diluent to melt or soften, whereby it forms agglomerates,
(b) breaking down the larger agglomerates to give controlled release seeds; and
(c) continuing mechanically working with optionally a further addition of low percentage of the carrier or diluent.
optionally repeating steps (c) and possibly (b) one or more times.

This process is capable of giving a high yield (over 80%) of particles in a desired size range, with a desired uniformity of release rate of betaine, preferably glycine betaine or salt thereof.
The resulting particles may be sieved to eliminate any over-or undersized material then formed into the desired dosage units by for example, encapsulation into hard gelatin capsules containing the required dose of the active substance or by compression into tablets.

In this method in accordance with the invention preferably all the betaine, preferably glycine betaine or salt thereof is added in step (a) together with a major portion of the hydrophobic fusible release control material used. Preferably the amount of fusible release control material added in step (a) is between 10% and 90% w/w of the total amount of ingredients added in the entire manufacturing operation, more preferably between 20% and 70% w/w.

Stage (a) of the process may be carried out in conventional high speed mixers with a standard stainless steel interior, e.g. a Collette Vactron 75 or equivalent mixer. The mixture is processed until a bed temperature about 40°C. or above is achieved and the resulting mixture acquires a cohesive granular texture, with particle sizes ranging from about 1-3 mm to fine powder in the case of non-aggregated original material. Such material, in the case of the embodiments described below, has the appearance of agglomerates which upon cooling below 40°C. have structural integrity and resistance to crushing between the fingers. At this stage the agglomerates are of an irregular size, shape and appearance.

The agglomerates are preferably allowed to cool. The temperature to which it cools is not critical and a temperature in the range room temperature to 37.degree. C. may be conveniently used.

The agglomerates are broken down by any suitable means, which will comminute oversize agglomerates and produce a mixture of powder and small particles preferably with a diameter under 2 mm. It is currently preferred to carry out the classification using a Jackson Crockatt granulator using a suitable sized mesh, or a Comil with an appropriate sized screen. It was found that if too small a mesh size is used in the aforementioned apparatus the agglomerates melting under the action of the beater or impeller will clog the mesh and prevent further throughput of mixture, thus reducing yield. A mesh size of 12 has been found adequate.

The classified material is returned to the high speed mixer and processing continued. It is believed that this leads to cementation of the finer particles into particles of uniform size range.

In one preferred form of the method of the invention processing of the classified materials is continued, until the hydrophobic fusible materials used begin to soften/melt and optionally additional hydrophobic fusible material is then added. Mixing is continued until the mixture has been transformed into particles of the desired predetermined size range.

In order to ensure uniform energy input into the ingredients in the high speed mixer it is preferred to supply at least part of the energy by means of microwave energy.

Energy may also be delivered through other means such as by a heating jacket or via the mixer impeller and chopper blades.

After the particles have been formed they are cooled or allowed to cool, and may then be sieved to remove any over or undersized material.
The resulting particles may be used to prepare dosage units in accordance with the invention in the form of e.g. tablets or capsules in manners known per se.

The inventor have also found that particles containing betaine, preferably glycine betaine or a salt thereof produced by a melt processing as described in application PCT/SE93/00225 and the process described and claimed in our prior unpublished UK application No. 9324045.5 filed on 23 Nov. 1993 as well as the process described herein are particularly useful for processing into the form of tablets.

The inventor found that by suitable selection of the materials used in forming the particles and in the tabletting and the proportions in which they are used, enables a significant degree of control in the ultimate dissolution and release rates of the betaine, preferably glycine betaine or salt thereof from the compressed tablets.

Usually, to form a tablet in accordance with the invention, particles prepared as described above will be admixed with tabletting excipients e.g. one or more or the standard excipients such as diluents, lubricants, binding agents, flow aids, disintegrating agents, surface active agents or water soluble polymeric materials.

Suitable diluents are e.g. microcrystalline cellulose, lactose and dicalcium phosphate. Suitable lubricants are e.g. magnesium stearate and sodium stearyl fumarate. Suitable binding agents are e.g. hydroxypropyl methyl cellulose, polyvidone and methyl cellulose.

Suitable disintegrating agents are starch, sodium starch glycolate, crospovidone and croscarmalose sodium. Suitable surface active are Poloxamer 188.RTM., polysorbate 80 and sodium lauryl sulfate. Suitable flow aids are talc colloidal anhydrous silica.

Suitable water soluble polymers are PEG with molecular weights in the range 1000 to 6000.

To produce tablets in accordance with the invention, particles produced in accordance with the invention may be mixed or blended with the desired excipient(s), if any, using conventional procedures, e.g. using a Y-Cone or bin-blender and the resulting mixture compressed according to conventional tabletting procedure using a suitable size tabletting mold. Tablets can be produced using conventional tabletting machines, and in the embodiments described below were produced on standard single punch F3 Manesty machine or Kilian RLE15 rotary tablet machine.

Generally speaking I find that even with such a highly water soluble active agent as betaine, preferably glycine betaine or salt thereof tablets formed by compression according to standard methods give very low release rates of the active ingredient e.g. corresponding to release over a period of greater than 24 hours, say more than 36. The inventor found that the release profile can be adjusted in a number of ways. For instance a higher loading of the drug will be associated with increased release rates; the use of larger proportions of the water soluble fusible material in the particles or surface active agent in the tabletting formulation will also be associated with a higher release rate of the active ingredient. By controlling the relative amounts of these ingredients it is possible to adjust the release profile of the betaine, preferably glycine betaine or salt thereof.

The following examples illustrate various aspects of the present invention. They are not to be construed to limit the claims in any manner whatsoever.

### APPARATUS AND METHOD

### MATERIAL

Betaine anhydrous, BETAFIN ® (Finnsugar Bioproducts, CULTOR, Helinski)
Rats Wistar, males, weight between 250 and 300 grams
Sodium Thiopental
Aggregometer CHRONOLOG COULTRONIC S.A. France
ADP & collagen Laboratoires Stago France

### METHODS

The aggregation is made in accordance to the methods Cardinal & Flower. Pharmacol. Method. 1980 . and to American Journal of Clinical Pathology, 1989; 92: 676-679. Sureney. JD. Whole Blood aggregometry.
After a keeping period of 8 days, the rats are subjected to a fasting for 12 hours. Betaine is subcutaneous injected one hour before blood sampling. The rats are then anaesthetised with sodium Thiopental administered at a dose of 200mg/Kg and the blood samples are taken by intracardiac puncture on a trisodium citrate solution (1 volume of solution at 3,8 % citrate for 9 volumes of blood).

### Activated coagulation time (kaolin)

This test explores the intrinsic coagulation pathway. One hour after sub cutaneous administration of the betaine, 0.8 ml total blood by intracardiac way is injected in a container HR, HemoTec. These tubes contain the kaolin activator. ( Method HemoTec., automated coagulation timer manufactured by MEDTRONIC HEMOTEC Inc., Englewood, CO., USA).

### Induced haemorrahgic time IHT

### ( E. Dejana. Bleeding time in rats. Thrombosis. Rech. 1982)

Blood samples are made before the test. The tail of anaesthetised rat, is dipped for 5 minutes in a water bath at 37°C so as to provoke a dilatation of the peripheral vessels which are removed and cut at the end, the chronometer being started. The IHT is defined as being the time period comprised between the cutting of end tail and the end of the haemorrhage or bleeding. The end of haemorrhage is defined as the time where the last drop of blood is removed from the tail and no other drop is seen during 180 seconds. The substances were subcutaneously administrated 60 minutes prior to the tail cut.
A/ Principle of laser-induced thrombosis
(Seiffge D. et al., 1989; Weichter W. et al., 1983)

In this model, lesion of the vascular wall is induced by a laser beam. This beam causes a limited lesion of the vascular endothelium (only 1 to 2 cells are destroyed). This laying bare of the sub-endothelium, which is a thrombogenetic surface, results in the adherence of platelets via glycoprotein II. This adherence of platelets is followed by the activation thereof. They form pseudopods and secrete the content of their granules. This activation results in the appearance of glycoproteins IIb-IIIa which are necessary for the aggregation of the platelets between them. This lesion is induced in the mesenteric microcirculation of the rat. It is immediately followed by the formation of a thrombus (in a few seconds). This thrombus, which rapidly enlarges under the influence of the flow of blood, embolises before being formed again.

By this manes, the assessment of the effect of glycine betaine was conducted pharmacologically in conjunction with the study of two active molecules used as a reference; namely acetylsalicylic acid and heparin (of low molecular weight). The assessment also involved the activity of glycine betaine in relation to the prothrombotic effects induced by contrasting products.

### B / Stasis-induced thrombosis

A laparotomy was performed to open the lower vena cava, on which a ligature was placed at To, followed by subcutaneous injection of glycine betaine at To + 2 hours, followed by withdrawal of the clot and blood samples at To + 6 hours.

### C/ Experimental procedure

Male Wistar rats were used for these tests. They weighed between 200 and 250 grams. After an 8-day stabilisation period, the rats were subjected to fasting for 12 hours. They were than anaesthetised, glycine betaine was administered subcutaneously, and the mesentery (laser) or vena cava (stasis) was opened at the end of the experiments.

### EXAMPLES:

### Example 1: Evaluation of the number of emboles and the duration of embolisation after vascular change due to laser firings.

| | **Number of emboles or embolies** | **Duration of embolisation (minutes)** |
|---|---|---|
| **Negative control NaCl 0.9%** | 5.33 ± 0.58 | 2 ± 0 |
| **Glycine betaine 5 mg/kg** | 2 ± 0 | 1 ± 0 |
| **Acetylsalicylic acid 100 mg/kg** | 1 ± 1 | 0.33 ± 0.58 |
| **Heparin 2 mg/kg** | 2.67 ± 0.52 | 1 ± 0 |

Glycine betaine considerably reduced the number of emboles and the duration of embolisation after vascular change due to laser firings. The results demonstrate its powerful anti-thrombotic activity.

### Example2: Evaluation of the bleeding time caused

### ( E. Dejana. Bleeding time in rats. Thrombosis. Rech. 1982)

| | **Bleeding time (seconds)** |
|---|---|
| **Negative control NaCl 0.9%** | 101.52±5.7 |
| **Glycine betaine 5 mg/kg** | 95 ± 5 |
| **Acetylsalicylic acid 100 mg/kg** | 276.67 ± 20.82 |
| **Heparin 2 mg/kg** | 313.33 ± 20 |

These results show that glycine betaine maintains the bleeding time which is caused within the values of the negative control. In addition to its anti-thrombotic activity, glycine betaine does not result in any risk of haemorrhage compared with the positive controls.

### Example 3: Evaluation of platelet aggregation after vascular change due to laser firings

### (Cardinal & Flower. Pharmacol. Method. 1980)

| | **Amplitude (ohms)** | **Velocity (ohms/min)** |
|---|---|---|
| **Negative control NaCl 0.9%** | 13 ± 1 | 9 ± 1 |
| **Glycine betaine 5 mg/kg** | 0.66 ± 1.15 | 1.66 ± 1.15 |
| **Acetylsalicylic acid 100 mg/kg** | 2.33 ± 2.08 | 2 ± 1 |
| **Heparin 2 mg/kg** | 4.33 ± 0.57 | 2.66 ± 0.50 |

These results demonstrate the anti-aggregation effect of glycine betaine.

### Example 4: Evaluation of the effect on blood cells

### a / Platelet count

| | **Number of platelets (10⁹)** |
|---|---|
| Negative control NaCl 0.9% | 788 ± 30.14 |
| **Glycine betaine 5 mg/kg** | 804.67 ± 20.03 |
| **Acetylsalicylic acid 100 mg/kg** | 855.33 ± 63.17 |
| **Heparin 2 mg/kg** | 777.33 ± 6.43 |

### b/ White corpuscle count

| | **Number of white corpuscles (10⁹)** |
|---|---|
| Negative control NaCl 0.9% | 5.03 ± 1 |
| **Glycine betaine 5 mg/kg** | 4.43 ± 0.32 |
| **Acetylsalicylic acid 100 mg/kg** | 4.33 ± 1.00. |
| **Heparin 2 mg/kg** | 5.80 ± 0.10 |

### c/ Red corpuscle count

| | **Number of red corpuscles (10¹²)** |
|---|---|
| Negative control NaCl 0.9% | 6.56 ± 0.15 |
| **Glycine betaine 5 mg/kg** | 6.19 ± 0.25 |
| **Acetylsalicylic acid 100 mg/kg** | 6.15 ± 0.31 |
| **Heparin 2 mg/kg** | 6.20 ± 0.20 |

The counts of the elements occurring in the blood remained within the values of the negative control and demonstrated the innocuousness of glycine betaine

### Example 5: Biological Balance

### a/ Quick time

| | **QT (seconds)** |
|---|---|
| Negative control NaCl 0.9% | 17 ± 1 |
| **Glycine betaine 5 mg/kg** | 16.9 ± 1.05 |
| **Acetylsalicylic acid 100 mg/kg** | 18.33 ± 2.08 |
| **Heparin 2 mg/kg** | 29.50 ± 0.52 |

### b / Activated cephaline time (ACT)

| | **ACT (seconds)** |
|---|---|
| Negative control NaCl 0.9% | 20.5 ± 0.5 |
| **Glycine betaine 5 mg/kg** | 39.9 ± 1.05 |
| **Acetylsalicylic acid 100 mg/kg** | 27.26 ± 1.1 |
| **Heparin 2 mg/kg** | 39.46 ± 1.36 |

### c/ Fibrinogen analysis

| | **Fibrinogen (g/l)** |
|---|---|
| Negative control NaCl 0.9% | 2.45 ± 0.19 |
| **Glycine betaine 5 mg/kg** | 1.7 ± 0.1 |
| **Acetylsalicylic acid 100 mg/kg** | 2.19 ± 0.33 |
| **Heparin 2 mg/kg** | 2.13 ± 0.25 |

### d/ Alpha,2-antiplasmin analysis (α2AP)

| | **α2AP(%)** |
|---|---|
| Negative control NaCl 0.9% | 30.16 ± 0.85 |
| **Glycine betaine 5 mg/kg** | 29.7 ± 0.68 |
| **Acetylsalicylic acid 100 mg/kg** | 29.36 ± 0.92 |
| **Heparin 2 mg/kg** | 29.4 ± 1.01 |

### e /Antithrombin III analysis (AT III)

| | **AT III (%)** |
|---|---|
| Negative control NaCl 0.9% | 86 ± 3 |
| **Glycine betaine 5 mg/kg** | 89.5 ± 1.37 |
| **Acetylsalicylic acid 100 mg/kg** | 85.33 ± 3.51 |
| **Heparin 2 mg/kg** | 77.66 ± 1.52 |

### Example 6: Evaluation of the activity of glycine betaine as a function of time

### Experimental groups: The product was tested at 5 mg/kg

### Laser-induced thrombosis

| Control | NaCl 0,9% |
|---|---|
| Group **I** | The product was injected 1 hour before the experiment |
| Group **II** | The product was injected 2 hours before the experiment |
| Group **III** | The product was injected 3 hours before the experiment |
| Group **IV** | The product was injected 4 hours before the experiment |

### a) Effect of the product tested (5 mg/ml/kg) on the bleeding time caused.

| **Group** | **B.T.C. (seconds)** |
|---|---|
| NaCl 0.9% | 110 ± 21.2 |
| **I** | 105 ± 26.2 |
| **II** | 145 ± 15.52 |
| **III** | 115.5 ± 14.2 |
| **IV** | 120 ± 10.13 |

### b) Effect of the product tested (5 mg/ml/kg) on arterial thrombosis induced by laser beam

| **Group** | **Number of firings** | **Number of emboles** | **Duration of embolisation (minutes)** |
|---|---|---|---|
| NaCl 0.9% | 2.5 ± 0.84 | 5.7 ± 1.5 | 2.1 ± 0.69 |
| **I** | 3.49 ± 1.07 | 1.8 ± 1.44 | 0.51 ± 0.5 |
| **II** | 3.0 ± 1.5 | 1.4±1.18 | 0.3 ± 0.23 |
| **III** | 2.50 ± 1.25 | 1.99 ± 0.4 | 1.00 ± 0.5 |
| **IV** | 2.7 ± 1.0 | 2.2 ± 0.69 | 1.5 ± 0.6 |

### c) Effect of the product tested (5 mg/kg) on platelet aggregation induced ex vivo.

| **Group** | **Amplitude (Ohms)** | **Velocity (ohm/minute)** |
|---|---|---|
| NaCl 0.9% | 24.23 ± 0.5 | 14.4 ± 2.3 |
| **I** | 11.33 ± 3.08 | 8.2 ± 0.2 |
| **II** | 13.2 ± 3.5 | 9.3 ± 1.8 |
| **III** | 12.7 ± 4.1 | 8.7 ± 1.3 |
| **IV** | 13 ± 2.8 | 8.7 ± 1.15 |

### d) Evaluation of the effect of glycine betaine on coagulation factors after repeated administration on 5 days of treatment

| | **ACT (seconds)** | **Quick time (seconds)** | **Fibrinogen g/l** |
|---|---|---|---|
| Untreated control | 21.25 ± 2.3 | 16.1 ± 1.0 | 3.03 ± 0.45 |
| **Glycine betaine (5 mg/kg/day)** | 39.3 ± 2.3 | 19.8 ± 1.2 | 2.2 ± 0.1 |

### Example 7: Evaluation of the effect of glycine betaine on venous thrombosis induced by stasis.

### a) Effect of glycine betaine on clot weight

| | **Clot weight (mg)** |
|---|---|
| Untreated control | 4.033 ± 2 |
| **Glycine betaine (1 mg/kg)** | 3.1 ± 0.4 |
| **Glycine betaine (2.5 mg/kg)** | 1.63 ± 0.76 |
| **Glycine betaine (5 mg/kg)** | 0.76 ± 0.4 |

### b) Evaluation of the effect of glycine betaine on plasminogenesis

| | **Plasminogenesis** % |
|---|---|
| NaCl 0.9% | 2.7 ± 0.33 |
| **Glycine betaine (5 mg/kg)** | 1.66 ± 0.58 |
| **Glycine betaine (2.5 mg/kg)** | 2 ± 0.15 |
| **Glycine betaine (1mg/kg)** | 2.44 ± 0.58 |

### c) Evaluation of the effect of glycine betaine on coagulation

| | **ACT (seconds)** | **Quick time (seconds)** | **Fibrinogen g/l** |
|---|---|---|---|
| Untreated control | 30.2 ± 2.7 | 16.1 ± 1.0 | 3.03 ± 0.45 |
| **Glycine betaine (1 mg/kg)** | 29.1 ± 2.3 | 16.2 ± 1.2 | 2.63 ± 0.3 |
| **Glycine betaine (2.5 mg/kg)** | 31.2 ± 2.6 | 16.6 ± 0.7 | 2.2 ± 0.17 |
| **Glycine betaine (5 mg/kg)** | 33.5 ± 1.9 | 15.6 ± 0.4 | 2.32 ± 0.33 |

### d) Evaluation of the effect of glycine betaine on coagulation factors

| | **Anti Xa units/ml** | **Anti IIa units/ml** |
|---|---|---|
| **Glycine betaine (5 mg/kg)** | 0.35 ± 0.15 | - |
| **Glycine betaine (2.5 mg/kg)** | 0.14 ± 0.10 | - |
| **Glycine betaine (1 mg/kg)** | 0.08 ± 0.1 | - |

Treatment with glycine betaine inhibits the thrombo-embolic complications which are initiated by laser firings. In fact, treatment with glycine betaine before laser firings decreases the vascular adherence of platelets and the aggregation thereof.

Treatment with glycine betaine inhibits thrombo-embolic complications. In fact, treatment with glycine betaine before the induction of thrombosis exhibited a high antithrombotic potential with regard to all the parameters which come into play in the process of thrombus formation. Moreover, the results for the biological parameters demonstrate the complete innocuousness of glycine betaine, which, in contrast to the reference products used (aspirin and heparin), does not induce any bleeding effect or undesirable side effect. These features mean that glycine betaine, in addition to its demonstrated efficacy, can be administered to people at risk of haemorrhage as well as to people who would be subject to risk of sensitivity or allergy if given conventional antithrombotic treatments (haemophiliac, allergic). Glycine betaine does not cause thrombopenia or haemorrahgic disorders (Examples 2 & 4). The experimental results of Example 5c shows that there is a consumption of fibrinogen.

It should be noted that, under the same experimental conditions for the preservation of blood, glycine betaine appeared to possess a high anticoagulant capacity compared with tubes containing heparin or EDTA. The effective dose of glycine betaine appeared to be between 3 and 5 mg per haemolysis tube. This experimental result demonstrates the high anticoagulant potential of glycine betaine. It can thus be claimed that glycine betaine can be used as an anticoagulant both for the treatment of the human body in vivo and for the preservation of blood ex vivo.

Glycine betaine possesses the same, or even better, therapeutic features as those of the anticoagulants and anti-aggregants investigated (acetylsalicylic acid and heparin), whilst exhibiting no undesirable effects.

The superior performance as regards therapeutic efficacy of glycine betaine in relation to these two molecules (acetylsalicylic acid and heparin) is an incentive for the formulation of a drug containing glycine betaine as a therapeutically active ingredient, said drug being intended for the treatment of thromboses and thrombo-embolic diseases.

According the results presented above, this drug also exhibits anticoagulant, anti-aggregant and fibrinolytic indications. The demonstrated innocuousness of this molecule enables long-term treatments to be considered which do not necessitate biological monitoring.

Interest in the use of glycine betaine is based on the fact that it acts at several levels of haemostatis, i.e. it acts on platelet aggregation, coagulation and fibrinolysis. This activity is durable and prevents repeated administration, which constitutes a considerable improvement in relation to existing treatments. The administration of betaine does not induce any haemorrahgic risk or other side effects (e.g. heparin-induced thrombopenia), which constitutes a major advance in antithrombotic therapy.

### Example 8 : Aggregation induced with ADP

### Final ADP concentration 5µM

| | Amplitude (ohms) | Velocity (ohm/minute) |
|---|---|---|
| Control NaCl 0.9% | 16.4 +/- 1.67 | 13.8 +/- 1.3 |
| Glycine betaine 2.5 mg/kg | 13 +/- 0.82 | 5.75 +/- 0.96 |
| Glycine betaine 5 mg/kg | 7.25 +/- 0.96 | 4.75 +/- 0.5 |
| Glycine betaine 10 mg/kg | 0+/-0 | 0 +/- 0 |

The dose effect of the betaine shows its action on the glycoprotein IIb IIIa site, the betaine competing in a dose dependant manner with the agonist (ADP).

### Example 9 : Aggregation induced with collagen

### Collagen concentration 10 µgr/ml

| | Amplitude (ohms) | Velocity (ohm/minute) |
|---|---|---|
| Control NaCl 0.9% | 16.75 +/- 0.96 | 9.75 +/- 0.98 |
| Glycine betaine 2.5 mg/kg | 13.75 +/- 0.96 | 9.25 +/- 2.63 |
| Glycine betaine 5mg/kg | 5.5 +/- 1.29 | 4.5 +/- 1 |
| Glycine betaine 10 mg/kg | 1.5 +/- 1.29 | 2 +/- 0.82 |

The dose effect of the betaine shows its action on the glycoprotein IIb IIIa site, the betaine competing in a dose dependant manner with the agonist (Collagen).

### Example 10 : Activated coagulation time (subcutaneous administration of betaine at different dosages)

| | Activated Coagulation time (seconds) | Provoked Haemorrhage time (seconds) |
|---|---|---|
| Control NaCl 0.9% | 48 | 107 |
| Glycine betaine 10 mg/kg | 128 | 105 |
| Glycine betaine 30 mg/kg | 179 | 112 |
| Glycine betaine 50 mg/kg | 215 | 115 |

The activated coagulation time is four time higher at a concentration of 50 mg/kg, while not having an effect on the PHT.

### Example 11 : Activated Coagulation Time after subcutaneous administration of betaine at 10 mg/kg after 24 hours

| | Provoked hemorrhage seconds | Activated coagulation time (seconds) |
|---|---|---|
| Control NaCl 0.9% | 105 +/-5 | 48.4 +/- 8.9 |
| Betaine 10mg/kg | 114 +/- 12.76 | 71 +/- 3.5 |

### Example 12 : Parameters of Thrombosis induced by laser 24 hours after sub cutaneous administration of betaine at 10 mg/kg

| | Number of laser firing | Number of emboles | Embolisation time (minutes) |
|---|---|---|---|
| Control NaCl 0.9% | 2.33 +/- 0.57 | 5.33 +/- 0.57 | 2 +/- 0 |
| Betaine 10mg/kg | 3.33 +/- 0.57 | 1 +/- 0 | 0 +/- 0 |

### Example 13 : Activated Coagulation Time after subcutaneous administration of betaine at 20 mg/kg after 24 hours

| | Provoked hemorrhage seconds | Activated coagulation time (seconds) |
|---|---|---|
| Control NaCl 0.9% | 105 +/- 5 | 48.4 +/- 8.9 |
| Betaine 20mg/kg | 110 +/- 13.22 | 154.66:+/-11.01 |

### Example 14 : Parameters of Thrombosis induced by laser 24 hours after sub cutaneous administration of betaine at 20 mg/kg

| | Number of laser firing | Number of emboles | Embolisation time (minutes) |
|---|---|---|---|
| Control NaCl 0.9% | 2.33 +/- 0.57 | 5.33 +/- 0.57 | 2+/-0 |
| Betaine 10mg/kg | 3.33 +/- 0.57 | 0.66 +/- 0.57 | 0 +/- 0 |

### Example 15 : Kinetic of the activated coagulation time after oral administration of betaine at 50mg/kg

| | Induced hemorrhage seconds | Activated coagulation time (seconds) |
|---|---|---|
| Control NaCl 0.9% | 105 +/- 5 | 48.4 +/- 8.9 |
| Betaine 50mg/kg- 1 hour | 120 +/- 5 | 96 +/-11.27 |
| Betaine 50mg/kg - 6 hours | 111 +/- 3.6 | 124.66 +/- 9.29 |
| Betaine 50mg/kg - 24 hours | 113.33 +/- 18.92 | 64.66 +/- 7.37 |
| Betaine 50mg/kg - 48 hours | 109 +/. 8.54 | 55.66 +/- 7.02 |

### Example 16 : Kinetic of Parameters of Thrombosis induced by laser and effect at different time for the oral administration of betaine at 50 mg/kg

| | Number of laser firing | Number of emboles | Embolisation time (minutes) |
|---|---|---|---|
| Control NaCl 0.9% | 2.33 +/- 0.57 | 5.33 +/- 0.57 | 2 +/- 0 |
| Betaine 50mg/kg 1 hour | 4+/-0 | 0+/-0 | 0+/-0 |
| Betaine 50mg/kg 6 hours | 3.66 +/- 0.57 | 2.33 +/- 0.57 | 1+/-0 |
| Betaine 50mg/kg 24 hours | 2.33 +/- 0.57 | 2.33 +/- 0.57 | 1 +/- 0 |
| Betaine 50mg/kg 48 hours | 2.33 +/- 0.57 | 4.33 +/- 0.57 | 2+/-0 |

The dose effect is confirmed in all the studied parameters. The antagonist activity at the glycoprotein IIb IIIa site for the compounds of the invention also applies to other agonists, such as serotinin, arachidonic acid, epinephrine, adrenaline, ristocetine and thrombin.

### Example 17 : Human in vivo test

Two volunteers (with a weight of about 70-75 kg), considered as heavy smokers (smoking more than 10 cigarettes / day) have orally taken capsules (gastro soluble) containing 5g anhydrous glycine betaine/ day during 7 days.

Before administration of the betaine, the aggregations induced by ADP, by collagen, by epinephrine, by adrenaline, by thrombin, by ristocetine and by arachidonic acid were determined. After one week treatment, the same platelet aggregations were measured. It appears from said tests that all the induced platelet aggregations were reduced for all said endogenous agonists of at least 30%.

Better results are expected when using oral dosage form with controlled release, such as gastro insoluble, but entero soluble form.

### Example 18 : anti-haemorrahgic activity

Rats have been used in this test. Some rats received an active agent with haemorrahgic side effect, while other rats received said active agent together with a dose of betaine.

The following products were administered to rats for determining whether glycine betaine has an anti-haemorrahgic effect:
100 mg aspirin per kg life body
100 mg aspirin + 50 mg glycine betaine per kg life body
2 mg heparin
2 mg heparin + 50 mg glycine betaine
2 mg heparin + 10 mg glycine betaine
2 mg heparin + 2 mg glycine betaine

When inducing a haemorrhage, it was observed that the bleeding time was reduced when glycine betaine was administered. It means therefore that glycine betaine has anti-haemorrahgic properties.

### EXAMPLE 19

Tablets having the following formulation were prepared:

| mg/tablet |
|---|
| Betaine, preferably glycine betaine Anhydrous 100 |
| Lactose Ph. Eur. 68.0 |
| Ethylcellulose (Surelease .RTM. 25% solids) |
| 15 |
| Purified Water Ph. Eur. |
| 13.3* |
| Cetostearyl Alcohol Ph. Eur. |
| 42.00 |
| (Dehydag wax 0) Magnesium Stearate Ph. Eur. |
| 2.00 |
| Purified Talc Ph. Eur. 3.00 |
| 230.00 |

| |
|---|
| *Removed during processing. |

Betaine, preferably glycine betaine anhydrous (100 mg) and lactose (68 mg) were granulated, transferred to a fluid bed granulator and sprayed with ethylcellulose (15 mg) and water. The granules were then dried at 60.degree. C. and passed through a 1 mm screen.

To the warmed betaine, preferably glycine betaine containing granules was added molten cetostearyl alcohol (42 mg) and the whole was mixed thoroughly. The granules were allowed to cool and sieved through a 1.6 mm screen. Purified talc and magnesium stearate were added and mixed with the granules. The granules were then compressed into tablets.

The tablets were coated with a film coat having the formulation given below.

| mg/tablet |
|---|
| Hydropropylmethylcellulose |
| 0.770 |
| Ph. Eur. 15 cps (Methocel E15) Hydroxypropylmethylcellulose |
| 3.87 |
| (Ph. Eur. 5 cps (Methocel E5) Opaspray M-1-7111B (33% solids) |
| 2.57 |
| Polyethylene glycol 400 USNF |
| 0.520 |
| Purified Talc Ph. Eur. |
| 0.270 |
| Purified Water Ph. Eur. |
| 55.52* |

| |
|---|
| *Remove during processing. |

### EXAMPLE 20

Tablets having the following formulation were prepared:

| mg/tablet |
|---|
| Betaine, preferably glycine betaine anhydrous |
| 100.0 |
| Lactose Ph. Eur. 58.0 |
| Ethylcellulose USNF 15.0 |
| (Ethocel 45 CP) |
| Cetostearyl alcohol Ph. Eur. |
| 52.0 |
| (Dehydag wax O) |
| Magnesium stearate Ph. Eur. |
| 2.00 |
| Purified talc Ph. Eur. |
| 3.00 |

A mixture of betaine, preferably glycine betaine anhydrous (100 mg), lactose (58 mg) and ethylcellulose (15 mg) was granulated while adding molten cetostearyl alcohol (52 mg) and the whole was mixed thoroughly. The granules were allowed to cool and sieved through a 1.6 mm screen. Purified talc and magnesium stearate were added and mixed with the granules. The granules were then compressed into tablets which were coated with a film coat having the formulation given in Example 24.

### EXAMPLE 21

Film coated tablets were produced following the procedure described in Example 25 and having the following formulation:

| mg/tablet |
|---|
| Betaine, preferably glycine betaine anhydrous |
| 100.00 |
| Lactose Ph. Eur. 70.50 |
| Hydroxyethylcellulose Ph. Eur. |
| 12.50 |
| Cetostearyl alcohol Ph. Eur. |
| 42.00 |
| Magnesium stearate Ph. Eur. |
| 2.00 |
| Purified talc Ph. Eur. |
| 3.00 |

### In vitro dissolution studies

In vitro dissolution studies were conducted on tablets prepared as described above. Results are given in Table 1.

**TABLE 1**

| WT % BETAINE, PREFERABLY GLYCINE BETAINE RELEASED | | | |
|---|---|---|---|
| Time (h) | Example 19 | Example 20 | Example 21 |
| 1 | 39 | 35 | 43 |
| 2 | 52 | 47 | 60 |
| 4 | 67 | 62 | 84 |
| 8 | 82 | 78 | 97 |
| 12 | 90 | 86 | -- |

| | | | |
|---|---|---|---|
| *Measured on tablet core | | | |

### EXAMPLES 22 and 23

Particles having the formulations given in Table II below, were prepared by the steps of:
i. Placing the ingredients (a) and (c) (total batch weight 0.7 kg) in the bowl of a 10 liter capacity Collette Gral Mixer (or equivalent) equipped with variable speed mixing and granulating blades;
ii. Mixing the ingredients at about 150-1000 rpm whilst applying heat until the contents of the bowl are agglomerated.
iii. Classifying the agglomerated material by passage through a Comil and/or Jackson Crockatt to obtain controlled release seeds.
iv. Warming and mixing the classified material in the bowl of a 10 liter Collette Gral, until uniform multiparticulates of the desired pre-determined size range are formed in yield of greater than 80%. This takes approximately 5 minutes.
v. Discharging the multiparticulates from the mixer and sieving them to separate out the multiparticulates collected between 0.5 and 2 mm aperture sieves.

**TABLE II**

| Example | 22 | 23 |
|---|---|---|
| (a) Betaine, preferably glycine betaine Anhydrous (Wt %) | 50 | 75 |
| (b) Hydrogenated Vegetable Oil (Wt%) | 50 | 25 |

### EXAMPLES 24

Samples of the particles from Example 22 were blended with magnesium stearate and purified talc using a Y-Cone or bin-blender. The blended mixture was then compressed using either (1) 14.times.6 mm, (2) 16.times.7 mm or (3) 18.6.times.7.5 mm capsule shaped tooling on a single punch F3 Manesty tabletting machine to give tablets giving 200, 300 and 400 mg of betaine, preferably glycine betaine anhydrous. The ingredients per dosage unit amounted to the following:

**TABLE III**

| TABLET MG/TABLET | | | |
|---|---|---|---|
| INGREDIENT | 24(1) | 24(2) | 24(3) |
| Betaine, preferably glycine betaine Anhydrous | 200 | 300 | 400 |
| Hydrogenated Vegetable Oil | 200 | 300 | 400 |
| Sub Total | 400 | 600 | 800 |
| Purified Talc | 12.63 | 18.95 | 25.26 |
| Magnesium Stearate | 8.42 | 12.63 | 16.84 |

The tablets were assessed by the dissolution using Ph. Eur. Paddle Method 100 rpm, 0.1N HCl.

To assess the non-compressed particles the Ph Eur. Paddle was replaced by a modified Ph Eur. Basket.

The results are shown in Table IV below;

**TABLE IV**

| HOURS AFTER | | | | |
|---|---|---|---|---|
| START OF TEST % BETAINE, PREFERABLY GLYCINE BETAINE | | | | |
| | Anhydrous RELEASED | | | |
| | Particles | Tablet 24(1) | tablet 24(2) | Tablet 24(3) |
| 1 | 54 | 16 | 15 | 15 |
| 2 | 68 | 23 | 20 | 21 |
| 3 | 76 | 28 | 25 | 25 |
| 4 | 82 | 32 | 28 | 28 |
| 6 | 89 | 40 | 35 | 35 |
| 8 | 93 | 46 | 41 | 40 |
| 10 | 96 | 50 | 45 | 45 |
| 12 | 98 | 55 | 49 | 49 |
| 16 | 100 | 63 | 57 | 56 |
| 20 | NR | 70 | 63 | NR |

These results confirm the effectiveness of the tabletting in reducing the release rate.

### EXAMPLE 25

Samples of the particles from Example 23 were then tabletted using a procedure similar to Example 21 and the ingredients per unit dosage amounted to:

**TABLE V**

| TABLET MG/TABLET | | | |
|---|---|---|---|
| INGREDIENT | 25(4) | 25(5) | 25(6) |
| Betaine, preferably glycine betaine Anhydrous | 200 | 300 | 400 |
| Hydrogenated Vegetable Oil | 66.7 | 100 | 133 |
| Sub Total | 266.7 | 400 | 533 |
| Purified Talc | 7.63 | 11.44 | 15.25 |
| Magnesium Stearate | 5.16 | 7.63 | 10.17 |

The tablets and samples of non-compressed multiparticulates (each sample containing 400 mg of betaine, preferably glycine betaine anhydrous) were assessed by the dissolution method also described above. The results are shown in Table VI below;

**TABLE VI**

| HOURS AFTER | Particles Tablet 25(4) | | Tablet 25(5) | Tablet 25(6) |
|---|---|---|---|---|
| START OF TEST % BETAINE, PREFERABLY GLYCINE BETAINE Anhydrous RELEASED | | | | |
| 1 | 77 | 43 | 40 | 42 |
| 2 | 92 | 64 | 55 | 56 |
| 3 | 98 | 75 | 65 | 66 |
| 4 | 100 | 83 | 72 | 73 |
| 6 | 102 | 94 | 83 | 84 |
| 8 | 102 | 100 | 91 | 91 |
| 10 | 102 | NR | 96 | 97 |

These results show that by increasing the loading of the highly water soluble betaine, preferably glycine in betaine anhydrous (75% w/w in this example compared with 50% w/w in Example 24) a significantly faster release rate of the active ingredient can be achieved.

### EXAMPLE 26

Example 22 was repeated but with the following formulation:

| |
|---|
| Betaine, preferably glycine betaine Anhydrous 200 mg/tablet |
| Hydrogenated Vegetable Oil |
| 163.0 mg/tablet |

The resulting multiparticulates were blended as described in Example 24 with the following;

| |
|---|
| Purified Talc 11.5 mg/tablet |
| Magnesium Stearate 7.66 mg/tablet |

The blend was then compressed as described in Example 24 but using 15 mm. times. 6.5 mm normal concave capsule shaped plain/plain punches.

The resulting tablets were then assessed by the dissolution method described above. The results are shown in Table V.

| HOURS AFTER START OF TEST | % BETAINE, PREFERABLY GLYCINE BETAINE Anhydrous RELEASED |
|---|---|
| 1 | 20 |
| 2 | 27 |
| 3 | 32 |
| 4 | 37 |
| 6 | 44 |
| 8 | 50 |
| 10 | 55 |
| 12 | 60 |
| 16 | 67 |
| 20 | 73 |
| 24 | 77 |

The examples provided above are not meant to be exclusive. Many other variation of the present invention would be obvious to those skilled in the art, and are contemplated to be within the scope of the appended claims.

The man skilled in the art can adjust the release rate of a patch so as to have an adapted betaine concentration in the blood, for a period of for example 6 hours, 12 hours, 24 hours, etc.

The patch structure can be as taught in US 4,911,916, US4,917,676, US 5,536,503 and US 5,486,362. In said patch structure, a betaine of formula (CH₃)₃N⁺(CH₂)ₙCOO⁻ with n an integer from 1 to 5, preferably glycine betaine or a pharmaceutically acceptable salt thereof, esters thereof, precursors thereof, and mixtures thereof is used as active agent instead of the proposed active agent. The drawings of said patents (especially figures 2 and 3 of US 5536503) are incorporated by reference for teaching the possible form and structure of the patch.

Transdermal patches have a variety of advantages including avoidance of the gastro-intestinal tract, sustained action which readily can be adjusted, self-administration and the ability to immediately discontinue dosage. The term transdermal patch is intended to include patches capable of being affixed to the skin of an individual and having a part or component capable of delivering an active agent (a betaine of formula (CH₃)₃N⁺(CH₂)ₙCOO⁻ with n an integer from 1 to 5, preferably glycine betaine or a pharmaceutically acceptable salt thereof, esters thereof, precursors thereof, mixtures thereof), in a controlled sustained release manner. Examples of types of patches useful in this invention include those having a diffusion layer matrix and/or multicompartmental type patches. These will be described below for glycine betaine as active agent. There are many transdermal patches known to those of ordinary skill in the art and well described in the prior art. One such patch useful involves a diffusion matrix layer that uses a reticulated macroporous polymeric foam as a framework for holding a viscoelastic glycine betaine-polymer mixture. The patch is for example a multi (4 or more)-layer, laminated composite that is adapted to be adhered to the skin. The outermost layer, backing layer, functions as the primary structural element of the device as well as serving as a protective covering to prevent the glycine betaine from being transmitted from the device via the outermost surface. Backing layer preferably is made of a sheet or film of a resilient elastomer of about 10-75 microns thick. Examples of such elastomers include polyether block amide copolymers, polyethylene methacrylate block copolymers, polyurethanes, silicon elastomers and the like.

The glycine betaine-containing matrix layer functions as a reservoir for glycine betaine (possibly an enhancer), and optionally a pressure sensitive adhesive. The framework of the matrix is a reticulated macroporous polymeric foam. Preferably the network is essentially completely open pores (90% or greater). The pore rating of the reticulated foam will normally be in the range of about 10-40 pores per linear centimeter and the density (unfilled) will typically be in the range of about 0.01 to 0.5 g/cm3. Suitable polymers from which such foam frameworks may be manufactured include polyurethanes and polyethylenes.
A pressure sensitive adhesive layer covers the exposed face of the matrix layer and a release liner covers the pressure sensitive adhesive. The pressure sensitive adhesive layer is a medical grade adhesive composition having a thickness normally between about 25 and 100 microns. An example of such an adhesive is polydimethylsiloxane (Dow Coming 355 medical grade adhesive).

The pores of the foam are wholly or partly filled with a viscoelastic hydrophobic betaine-permeable polymer (and an enhancer if present). The polymer acts as a carrier for the glycine betaine, while the enhancer acts to control the solubility of the glycine betaine in the polymer and/or absorption of the drug into the skin. The hydrophobic polymer renders the device water-resistant and prevents liquid water from being absorbed by the device, thereby increasing its functionality and wearability. Examples of such polymers are polysiloxanes (silicone polymers), hydrophobic polyacrylates, polyurethanes, plasticized ethylene-vinyl acetate copolymers and the like. An example of a useful enhancer includes Azone. TM.. Another example of a useful dermal permeation enhancer includes transcutol. The mixture including the glycine betaine optionally includes an anti-pruritic agent.

Devices of the foregoing nature are generally described in U.S. Pat. No. 4,911,916, entitled "Diffusion Matrix for Transdermal Drug Administration and Transdermal Drug Delivery Devices Including Same", issued Mar. 27, 1990. Such patches may be configured to contain sufficient glycine betaine to release from about five milligrams to 5 grams of glycine betaine, such as from 100 to 500 milligrams per day. Preferably such patches are configured to hold sufficient betaine to release from about five to about 500 milligrams per day for seven days, such that a single patch may be worn for one week.

The optimum dose range, i.e., the range of doses with which the drug exhibits maximum therapeutic effect (and minimum adverse side effects for the other drug or therapeutic agent of the combination, when the patch is used in such a combination) can be determined empirically. The patch or other delivery system is configured and formulated to contain sufficient glycine betaine to release a dose within the optimum dose range for the desired period of time.

Another patch useful is a 4-layer composite defining at least two separate compartments. One compartment contains glycine betaine, and the other compartment contains a delivery substance that when mixed with glycine betaine permits the delivery of the glycine betaine transdermally. The patch has a backing layer sealed to a rate controlling membrane in a manner to create two chambers, betaine containing chamber and a delivery substance containing chamber. An adhesive layer covers the rate controlling membrane and a release sheet covers the adhesive layer.

To form the device, a silanized polyester (or other suitable material treated with a releasing agent) approximately 75 microns thick, is used as a release sheet 34. The adhesive layer 32 is cast onto the release sheet, and may be for example polyisobutylene. The adhesive layer then is laminated to the rate controlling membrane 26, which may be about 100 microns thick. Ethylene-vinyl acetate may be employed for the control membrane.

Next, the materials which will become the contents of the betaine containing chamber and delivery substance chamber are placed in separate areas on the rate controlling membrane. The material for the betaine containing chamber may be betaine free base and the material for the delivery substance containing chamber may be an alcoholic or aqueous/alcoholic solution or water.

Finally, a suitable backing having a heat sealable coating on one surface is placed over the two areas which are to become the two chambers, and the device is heat sealed around the perimeter and between the two areas to form the two chambers. The heat seal between the two chambers should be less secure than the heat seal about the perimeter, so that the seal between the chambers will selectively burst under pressure applied by the user. In this manner, pressure may be applied to either one of the chambers to burst the seal between the chambers, thereby mixing the solution and the betaine and dissolving the glycine betaine (anhydrous). The betaine then is in a form which is capable of passing through the rate controlling membrane for delivery to the skin of the user. As with the patch disclosed in the first embodiment, the chambers may include enhancers or retarder for affecting uptake of the betaine across the skin.

Preferred forms of the foregoing patch are shown in greater detail in U.S. Pat. No. 4,917,676, issued Apr. 17, 1990 and entitled "User-Activated Transdermal Therapeutic System". Such patches should contain sufficient lobeline to release from about five to about 500 milligrams of glycine betaine per day (from 2 to 10 mg/kg), and such patches are suitable to provide individual, daily patches.

### Example 27

In said example parenteral (especially subcutaneous) solutions containing heparin and glycine betaine have been prepared.
For example, the solutions of heparin sodium sold by CHOAY® (25000 IU/ml, aqueous solution), sold by LEO® and sold by ROCHE® were mixed with glycine betaine as a powder or as an aqueous solution, so as to prepare injectable solutions containing 25000 IU, 5000 IU and 2500 IU heparin (corresponding to 5 ml injectable solutions containing 5 mg /ml heparin and 100 mg/ml glycine betaine, injectable solutions containing 1 mg/ml heparin and 20 mg/ml glycine betaine, and injectable solutions containing 0,5 mg/ml heparin and 10 mg/ml glycine betaine).

### Example 28

In said example oral formulations containing aspirin (acid acetylsalicylic) and glycine betaine have been prepared.
A) acid acetylsalicylic 500 mg + 500 mg betaine + excipient
B) acid acetylsalicylic 300 mg + 200 mg betaine + excipient
C) acid acetylsalicylic 300 mg + 400 mg betaine + excipient
   A, B and C being possibly coated with an enterosoluble or controlled release layer, such as tablet or pellet Or A, B and C being possibly placed in a capsule with an enterosoluble or controlled release layer.
D) a syrup containing acid acetylsalicylic + betaine.

### Example 29

### Induced haemorrahgic time IHT

### (E. Dejana. Bleeding time in rats. Thrombosis. Rech. 1982)

Male Wistar rats were used for these tests. They weighed between 240 and 260 grams. The effect of various solutions (NaCl 0,9%, betaine anhydrous 10 mg/kg, heparin sodium CHOAY® 5 mg/ kg, combination betaine anhydrous 10 mg/kg + heparin CHOAY® 5 mg/ kg) on the (IHT) or induced haemorrahgic time was tested.
The tail of anaesthetised rat, is dipped for 5 minutes in a water bath at 37°C, so as to provoke a dilatation of the peripheral vessels, which are removed and cut at the end (6 to 10 mm from the end of the tail), the chronometer being started. The IHT is defined as being the time period comprised between the cutting of end tail and the end of the haemorrhage or bleeding. The end of haemorrhage is defined as the time where the last drop of blood is removed from the tail and where no other drop is seen during 180 seconds. The substances were subcutaneously administrated 60 minutes prior to the tail cut. In the combination test betaine 10 mg/ kg and heparin 5 mg/ kg were simultaneously administrated.

The results of said test are given in seconds in the following table.

| | **Reference NaCl 0,9%** | **Betaine 10 mg/kg** | **Heparin 5 mg/kg** | **Combination Betaine 10 mg + Heparin 5 mg** |
|---|---|---|---|---|
| Rat N° 1 | 110 | 162 | 767 | 218 |
| Rat N° 2 | 110 | 173 | 680 | 176 |
| Rat N° 3 | 156 | 194 | 734 | ** |
| Rat N° 4 | 140 | 203 | 702 | 231 |
| Rat N° 5 | 165 | 159 | ** | 229 |
| RatN°6 | 180 | 142 | 663 | 155 |
| Rat N° 7 | 106 | 123 | 735 | 278 |
| Rat N° 8 | 156 | 197 | 567 | 192 |
| Rat N° 9 | 97 | 166 | ** | 202 |
| Rat N° 10 | * | 180 | 679 | 257 |
| | n=9 | n=10 | n=8 | n=9 |
| **mean** | **135,6** | **169,9** | **690,9** | **215,3** |
| **Standard deviation** | 30,3 | 25,2 | 61,1 | 38,7 |

| | | | | |
|---|---|---|---|---|
| * rat dead after the anesthesia ** not measured ( no haemorrhage 3 minutes after cutting the end tail) | | | | |

This test shows clearly that the combined use of heparin with betaine prevent and reduces drastically the haemorrahgic side effect due to heparin. The simultaneous administration of betaine with heparin reduced in a statistically significant manner the volume of blood loss comparatively to the group where heparin was administrated alone. It was noted a reduction by more than 3 folds of blood loss in the combination group comparatively to the group where heparin was administrated alone.

## Claims

1. A pharmaceutical antithrombotic combination comprising : (a) a therapeutic effective amount of a therapeutically antithrombotic active agent causing at least one haemorrhagic side effect, said active agent being selected from the group consisting of :
anti aggregants selected from the group consisting of
abciximab, acetylsalicylate basic aluminium, acetylsalicylate carbonate sodium, acetylsalicylate lysine, acetylsalicylic acid, aloxiprine, anagreli chlorydrate, bencyclane furamate, carbasalate calcium, clopidogrel sulfate, epoprostenol sodium, epifibati, hydroxychloroquine sulfate, iloprost, nicergoline, nifepidine, pyricarbate, sulfinpyrazone, ticlopidine chlorhydrate, tirofiban chlorhydrate, verapamil chlorhydrate, and mixtures thereof,
and/ or anticoagulants selected from the group consisting of
acenocoumarol, anisindione, biscoumacetate ethyl, bromindione, coumetarol, sirudine, oxazidione, phenindione, phenprocoumone, tioclomarol, warfarine sodium, and mixtures thereof,
and/ or fibrinolytics selected from the group consisting of
altepase, anistreplase, atorvastatine calcium, bromelaines, ciprofibrate, defibrotide, fluvastatine sodium, glicazide, lovastatine, lys-plasminogene, phenformine, pravastatine sodium, reteplase, simvastatine, streptokinase, urokinase, and mixtures thereof ,
and/or thrombin inhibitor selected from the group consisting of argatroban, novastan, and mixtures thereof,
and/or anti vitamin K,
and mixtures thereof,
and (b) a therapeutic effective amount of a compound of formula (CH₃)₃N⁺(CH₂)ₙCOO⁻ with n an integer from 1 to 5, or a pharmaceutically acceptable salt thereof, or mixtures thereof, for preventing or reducing said haemorrhagic side effect and for potentialising the therapeutic antithrombotic effect of said active agent.

2. The pharmaceutical combination of claim 1, in which the therapeutic active agent has at least possible haemorrhagic side effects, and in which the combination comprises a therapeutic effective amount of glycine betaine for preventing substantially or completely said haemorrhagic side effect.

3. The pharmaceutical combination of claim 1, in which glycine betaine is in a form suitable for subcutaneous injection or in a form suitable for the preparation of a form for subcutaneous injection.

4. The pharmaceutical combination of claim 1, in which the therapeutic agent is selected from the group consisting of anti vitamin K, antiaggregants, anticoagulants, anti thrombin, fibrinolytics and mixtures thereof.

5. The pharmaceutical combination of claim 1, in which the therapeutic active agent which is an antithrombotic agent and the glycine betaine are in a form suitable for simultaneous administration or successive administration or for administration according to different paths.

6. The pharmaceutical combination of claim 1, in which the compound of formula (CH₃)₃N⁺(CH₂)ₙCOO⁻ is glycine betaine.

7. Use of a compound of formula (CH₃)₃N⁺(CH₂)ₙCOO⁻ with n an integer from 1 to 5, or a pharmaceutically acceptable salt thereof, mixtures thereof, as active antidote agent for the preparation of an antidote composition for therapeutically preventing side effects bound to an active agent being selected from the group consisting of:
anti aggregants selected from the group consisting of
abciximab, acetylsalicylate basic aluminium, acetylsalicylate carbonate sodium, acetylsalicylate lysine, acetylsalicylic acid, aloxiprine, anagreli chlorydrate, bencyclane furamate, carbasalate calcium, clopidogrel sulfate, epoprostenol sodium, epifibati, hydroxychloroquine sulfate, iloprost, nicergoline, nifepidine, pyricarbate, sulfinpyrazone, ticlopidine chlorhydrate, tirofiban chlorhydrate, verapamil chlorhydrate, and mixtures thereof,
and/ or anticoagulants selected from the group consisting of
acenocoumarol, anisindione, biscoumacetate ethyl, bromindione, coumetarol, dalteparine sodium, sirudine, xtran sulfate, enoxaparine sodium, fluindione, heparinate magnesium, heparin calcium, heparine sodium, lepirudine nadroparine calcium, oxazidione, pentosane polyester sulfuric, phenindione, phenprocoumone, reviparine sodium, tinzaparine sodium, tioclomarol, warfarine sodium, glycoaminoglycans, heparins, unfractioned heparin, standard heparin, low molecular heparins, heparinoids, heparin-like molecules, and mixtures thereof,
and/ or fibrinolytics selected from the group consisting of
altepase, anistreplase, atorvastatine calcium, bromelaines, ciprofibrate, defibrotide, fluvastatine sodium, glicazide, lovastatine, lys-plasminogene, phenformine, pravastatine sodium, reteplase, simvastatine, streptokinase, urokinase, and mixtures thereof,
and/or thrombin inhibitor selected from the group consisting of argatroban, novastan, and mixtures thereof,
and/or anti vitamin K,
and mixtures thereof.

8. The use of claim 7, in which the compound of formula (CH₃)₃N⁺(CH₂)ₙCOO⁻ is glycine betaine.

9. Use of glycine betaine as therapeutic active agent for the preparation of a pharmaceutical composition for treating or preventing a trouble caused by administering to a patient an effective amount of a therapeutic agent with at least one side haemorrhagic side effect, and/ or for potentializing the therapeutic effect of said therapeutic agent with at least one side hemorrhagic side effect.

10. Use of glycine betaine for the preparation of a pharmaceutical subcutaneous injectable composition for treating or for preventing haemorrhagic troubles in a patient, caused by administering to said patient a therapeutic effective amount of an antithrombotic active agent with at Least one haemorrhagic side effect.

11. A controlled release pharmaceutical system suitable for delivering after administration in a time-controlled manner to the bloodstream of a mammalian a glycine betaine or an effective amount of a compound of formula (CH₃)₃N⁺(CH₂)ₙCOO⁻ with n equal to 1 or a pharmaceutically acceptable salt thereof or mixtures thereof.

12. The system of the preceding claim, which is an oral controlled release preparation or device.

13. A controlled release pharmaceutical system according to claim 11, said system being a transdermal controlled release preparation or device, especially a patch.

14. The system of any one of the claims 11 to 13 for releasing as active ingredient glycine betaine.

15. An oral controlled release pharmaceutical system according to claim 11 for releasing in a time controlled manner for at least 120 minutes, after administration, an effective therapeutically amount of at least a glycine betaine or an effective amount of a compound of formula (CH₃)₃N+(CH₂)ₙCOO⁻ with n equal to 1, pharmaceutically acceptable salts thereof or mixtures thereof, for treating or preventing blood flow disturbances.

16. A controlled release pharmaceutical system according to claim 11 for releasing in a time controlled manner for at least 120 minutes, after administration, an effective therapeutically amount of a glycine betaine or an effective amount of a compound of formula (CH₃)₃N⁺(CH₂)ₙCOO⁻ with n equal to 1, pharmaceutically acceptable salts thereof or mixtures thereof, for treating or preventing thrombosis and/or thromboembolic disorders.

17. A controlled release pharmaceutical system according to claim 11 for releasing an effective therapeutic amount of at least a compound selected from the group consisting of betaines, pharmaceutically acceptable salts thereof or mixtures thereof, in which the system controls at least for 120 minutes the release of at least a glycine betaine or an effective amount of a compound of formula (CH₃)₃N⁺(CH₂)ₙCOO⁻ with n equal to 1, pharmaceutically acceptable salts thereof or mixtures thereof.

18. The system of claim 17, in which the system controls at least for 180 minutes the release of at least a glycine betaine or an effective amount of a compound of formula (CH₃)₃N⁺(CH₂)ₙCOO⁻ with n equal to 1, pharmaceutically acceptable salts thereof or mixtures thereof.

19. The system of claims 17 or 18, in which the system controls at least for 240 minutes, advantageously at least for 360 minutes, preferably at least for 2160 minutes, the release of at least a glycine betaine or an effective amount of a compound of formula (CH₃)₃N⁺(CH₂)ₙCOO⁻ with n equal to 1, pharmaceutically acceptable salts thereof, esters thereof or mixtures thereof.

20. The system of any one of the claims 11 to 19, in which the system comprises one or more electronic device or chips controlling one or more releasing system or device.

21. Use of glycine betaine as active agent for the preparation of a pharmaceutical system controlled release for treating or preventing a blood flow disturbance and/or thrombosis and/or thromboembolic disorders.

22. Use according to claim 21 for the transdermal administration of glycine betaine for treating or preventing a blood flow disturbance and/or thrombosis and/or thromboembolic disorders.

23. A pharmaceutical combination for oral, parenteral or rectal administration comprising : (a) a therapeutic effective amount of a therapeutically active agent causing at least one haemorrhagic side effect, said active agent being selected from the group consisting of :
dalteparine sodium, sirudine, xtran sulfate, enoxaparine sodium, fluindione, heparinate magnesium, heparin calcium, beparine sodium, lepirudine nadroparine calcium, pentosane polyester sulfuric, reviparine sodium, tinzaparine sodium, glycoaminoglycans, heparins, unfractioned heparin, standard heparin, low molecular heparins, heparinoids, heparin-like molecules, and mixtures thereof and
(b) a therapeutic effective amount of a compound of formula (CH₃)₃N⁺(CH₂)ₙCOO⁻ with n an integer from 1 to 5, pharmaceutically acceptable salt thereof, or mixtures
thereof, for preventing or reducing said haemorrhagic side effect and/or for potentialising the therapeutic effect of said active agent.

24. The pharmaceutical combination of claim 23, in which compound of formula (CH₃)₃N⁺(CH₂)ₙCOO⁻ is glycine betaine.

25. The pharmaceutical combination of claim 23, in which glycine betaine is in a form suitable for subcutaneous injection or in a form suitable for the preparation of a form for subcutaneous injection.

26. The pharmaceutical combination of claim 23, in which the therapeutic active agent which is an antithrombotic agent with side effects and the glycine betaine are in a form suitable for simultaneous administration or successive administration or for administration according to different paths.

## Patentansprüche

1. Pharmazeutische antithrombotische Kombination, umfassend:
(a) eine therapeutische wirksame Menge eines therapeutisch antithrombotischen Wirkstoffs, der mindestens eine hämorrhagische Nebenwirkung verursacht, wobei der Wirkstoff ausgewählt ist aus der Gruppe, bestehend aus:
Antiaggregantien, die ausgewählt sind aus der Gruppe, bestehend aus
Abciximab, basischem Aluminiumacetylsalicylat, Acetylsalicylatcarbonatnatrium, Lysin-Acetylsalicylat, Acetylsalicylsäure, Aloxiprin, Anagrelid-Hydrochlorid, Bencyclanfumarat, Carbasalat-Calcium, Clopidogrelsulfat, Epoprostenol-Natrium, Epifibati, Hydroxychloroquinsulfat, Iloprost, Nicergolin, Nifepidin, Pyricarbat, Sulfinpyrazon, Ticlopidin-Hydrochlorid, Tirofiban-Hydrochlorid, Verapamil-Hydrochlorid und Gemischen davon,
und/oder Antikoagulantien, die ausgewählt sind aus der Gruppe, bestehend aus
Acenocoumarol, Anisindion, Ethyl-Biscoumacetat, Bromindion, Coumetarol, Sirudin, Oxazidion, Phenindion, Phenprocoumon, Tioclomarol, Warfarin-Natrium und Gemischen davon,
und/oder Fibrinolytika, die ausgewählt sind aus der Gruppe, bestehend aus
Altepase, Anistreplase, Atorvastatin-Calcium, Bromelainen, Ciprofibrat, Defibrotid, Fluvastatin-Natrium, Glicazid, Lovastatin, Lys-Plasminogen, Phenformin, Pravastatin-Natrium, Reteplase, Simvastatin, Streptokinase, Urokinase und Gemischen davon,
und/oder einem Thrombininhibitor, der ausgewählt ist aus der Gruppe, bestehend aus Argatroban, Novastan und Gemischen davon,
und/oder Antivitamin K,
und Gemischen davon,
und (b) eine therapeutische wirksame Menge einer Verbindung der Formel (CH₃)₃N⁺(CH₂)ₙCOO⁻, wobei n eine ganze Zahl von 1 bis 5 ist, oder eines pharmazeutisch annehmbaren Salzes davon, oder von Gemischen davon zur Prävention oder Reduktion dieser hämorrhagischen Nebenwirkung und zum Verstärken der therapeutischen antithrombotischen Wirkung des Wirkstoffs.

2. Pharmazeutische Kombination nach Anspruch 1, wobei der therapeutische Wirkstoff zumindest mögliche hämorrhagische Nebenwirkungen besitzt und wobei die Kombination eine therapeutische wirksame Menge eines Glycinbetains zur wesentlichen oder vollständigen Prävention der hämorrhagischen Nebenwirkung umfasst.

3. Pharmazeutische Kombination nach Anspruch 1, wobei das Glycinbetain in einer Form, die zur subkutanen Injektion geeignet ist, oder in einer Form, die zur Herstellung einer Form zur subkutanen Injektion geeignet ist, vorliegt.

4. Pharmazeutische Kombination nach Anspruch 1, wobei das therapeutische Mittel ausgewählt ist aus der Gruppe, bestehend aus Antivitamin K, Antiaggregantien, Antikoagulantien, Antithrombin, Fibrinolytika und Gemischen davon.

5. Pharmazeutische Kombination nach Anspruch 1, wobei das therapeutische aktive Mittel, das ein antithrombotisches Mittel ist, und das Glycinbetain in einer Form vorliegen, die für die gleichzeitige Verabreichung oder die aufeinanderfolgende Verabreichung oder für die Verabreichung auf verschiedenen Wegen geeignet ist.

6. Pharmazeutische Kombination nach Anspruch 1, wobei die Verbindung der Formel (CH₃)₃N⁺(CH₂)ₙCOO⁻ Glycinbetain ist.

7. Verwendung einer Verbindung der Formel (CH₃)₃N⁺(CH₂)ₙCOO⁻, worin n eine ganze Zahl von 1 bis 5 ist, oder eines pharmazeutisch annehmbaren Salzes davon oder von Gemischen davon als aktives Gegenmittel zur Herstellung einer Gegenmittelzusammensetzung zur therapeutischen Prävention von Nebenwirkungen, die mit einem Wirkstoff verbunden sind, der ausgewählt ist aus der Gruppe, bestehend aus:
Antiaggregantien, die ausgewählt sind aus der Gruppe, bestehend aus:
Abciximab, basischem Aluminiumacetylsalicylat, Acetylsalicylatcarbonatnatrium, Lysin-Acetylsalicylat, Acetylsalicylsäure, Aloxiprin, Anagrelid-Hydrochlorid, Bencyclanfumarat, Carbasalat-Calcium, Clopidogrelsulfat, Epoprostenol-Natrium, Epifibati, Hydroxychloroquinsulfat, Iloprost, Nicergolin, Nifepidin, Pyricarbat, Sulfinpyrazon, Ticlopidin-Hydrochlorid, Tirofiban-Hydrochlorid, Verapamil-Hydrochlorid und Gemischen davon,
und/oder Antikoagulantien, die ausgewählt sind aus der Gruppe, bestehend aus
Acenocoumarol, Anisindion, Ethyl-Biscoumacetat, Bromindion, Coumetarol, Dalteparin-Natrium, Sirudin, Xtransulfat, Enoxaparin-Natrium, Fluindion, Heparinat-Magnesium, Heparin-Calcium, Heparin-Natrium, Lepirudin, Nadroparin-Calcium, Oxazidion, Pentosan-Polyestersulfat, Phenindion, Phenprocoumon, Reviparin-Natrium, Tinzaparin-Natrium, Tioclomarol, Warfarin-Natrium, Glycoaminoglycanen, Heparinen, unfraktioniertem Heparin, Standardheparin, niedermolekularen Heparinen, Heparinoiden, heparinartigen Molekülen und Gemischen davon,
und/oder Fibrinolytika, die ausgewählt sind aus der Gruppe, bestehend aus
Altepase, Anistreplase, Atorvastatin-Calcium, Bromelainen, Ciprofibrat, Defibrotid, Fluvastatin-Natrium, Glicazid, Lovastatin, Lys-Plasminogen, Phenformin, Pravastatin-Natrium, Reteplase, Simvastatin, Streptokinase, Urokinase und Gemischen davon,
und/oder einem Thrombininhibitor, der ausgewählt ist aus der Gruppe, bestehend aus Argatroban, Novastan und Gemischen davon,
und/oder Antivitamin K,
und Gemischen davon.

8. Verwendung nach Anspruch 7, wobei die Verbindung der Formel (CH₃)₃N⁺(CH₂)ₙCOO⁻ Glycinbetain ist.

9. Verwendung von Glycinbetain als therapeutischer Wirkstoff zur Herstellung einer pharmazeutischen Zusammensetzung zur Behandlung oder Prävention von Beschwerden, die durch Verabreichen einer wirksamen Menge eines therapeutischen Mittels mit mindestens einer hämorrhagischen Nebenwirkung an einen Patienten verursacht worden sind, und/oder zur Verstärkung des therapeutischen Effekts des therapeutischen Mittels mit mindestens einer hämorrhagischen Nebenwirkung.

10. Verwendung von Glycinbetain zur Herstellung einer therapeutischen subkutanen injizierbaren Zusammensetzung zur Behandlung und zur Prävention hämorrhagischer Beschwerden bei einem Patienten, die durch Verabreichen einer therapeutischen wirksamen Menge eines antithrombotischen Wirkstoffs mit mindestens einer hämorrhagischen Nebenwirkung an den Patienten verursacht worden sind.

11. Pharmazeutisches System zur kontrollierten Freisetzung, das zur Abgabe eines Glycinbetains oder einer wirksamen Menge der Formel (CH₃)₃N⁺(CH₂)ₙCOO⁻, wobei n gleich 1 ist, oder eines pharmazeutisch annehmbaren Salzes davon oder von Gemischen davon in die Blutbahn eines Säugetiers in einer zeitlich kontrollierten Art und Weise nach Verabreichung geeignet ist.

12. System des voranstehenden Anspruches, das eine orale Zubereitung oder Vorrichtung zur kontrollierten Freisetzung ist.

13. Pharmazeutisches System zur kontrollierten Freisetzung nach Anspruch 11, wobei das System eine transdermale Zubereitung oder Vorrichtung zur kontrollierten Freisetzung ist, insbesondere ein Pflaster.

14. System nach einem der Ansprüche 11 bis 13 zur Freisetzung von Glycinbetain als aktiven Inhaltsstoff.

15. Orales pharmazeutisches System zur kontrollierten Freisetzung nach Anspruch 11 zur Freisetzung einer therapeutisch wirksamen Menge mindestens eines Glycinbetains oder einer wirksamen Menge einer Verbindung der Formel (CH₃)₃N⁺(CH₂)ₙCOO⁻, wobei n gleich 1 ist, pharmazeutisch annehmbarer Salze davon oder von Gemischen davon in einer zeitlich kontrollierten Art und Weise für mindestens 120 Minuten nach Verabreichung zur Behandlung und/oder Prävention von Blutflussstörungen.

16. Pharmazeutisches System zur kontrollierten Freisetzung nach Anspruch 11 zur Freisetzung einer therapeutisch wirksamen Menge eines Glycinbetains oder einer wirksamen Menge einer Verbindung der Formel (CH₃)₃N⁺(CH₂)ₙCOO⁻, worin n gleich 1 ist, pharmazeutisch annehmbarer Salze davon oder von Gemischen davon in einer zeitlich kontrollierten Art und Weise für mindestens 120 Minuten nach Verabreichung zur Behandlung oder Prävention einer Thrombose und/oder thromboembolischer Erkrankungen.

17. Pharmazeutisches System zur kontrollierten Freisetzung nach Anspruch 11 zur Freisetzung einer wirksamen therapeutischen Menge mindestens einer Verbindung, die ausgewählt ist aus der Gruppe, bestehend aus Betainen, pharmazeutisch annehmbaren Salzen davon oder Gemischen davon, wobei das System zumindest 120 Minuten lang die Freisetzung mindestens eines Glycinbetains oder einer wirksamen Menge einer Verbindung der Formel (CH₃)₃N⁺(CH₂)ₙCOO⁻, worin n gleich 1 ist, pharmazeutisch annehmbarer Salze davon oder von Gemischen davon kontrolliert.

18. System nach Anspruch 17, wobei das System mindestens 180 Minuten lang die Freisetzung mindestens eines Glycinbetains oder einer wirksamen Menge einer Verbindung der Formel (CH₃)₃N⁺(CH₂)ₙCOO⁻, worin n gleich 1 ist, pharmazeutisch annehmbarer Salze davon oder von Gemischen davon kontrolliert.

19. System nach den Ansprüchen 17 oder 18, wobei das System mindestens 240 Minuten lang, vorteilhafterweise mindestens 360 Minuten lang, vorzugsweise mindestens 2.160 Minuten lang die Freisetzung mindestens eines Glycinbetains oder einer wirksamen Menge einer Verbindung der Formel (CH₃)₃N⁺(CH₂)ₙCOO⁻, worin n gleich 1 ist, pharmazeutisch annehmbarer Salze davon, Estern davon oder von Gemischen davon kontrolliert.

20. System nach einem der Ansprüche 11 bis 19, wobei das System eine oder mehr elektronische Vorrichtungen oder Chips umfasst, die ein(e) Freisetzungssystem oder -vorrichtung oder mehr kontrolliert/kontrollieren.

21. Verwendung von Glycinbetain als Wirkstoff zur Herstellung eines pharmazeutischen Systems zur kontrollierten Freisetzung zur Behandlung oder Prävention einer Blutflussstörung und/oder einer Thrombose und/oder thromboembolischer Erkrankungen.

22. Verwendung nach Anspruch 21 zur transdermalen Verabreichung von Glycinbetain zur Behandlung oder Prävention einer Blutflussstörung und/oder einer Thrombose und/oder thromboembolischer Erkrankungen.

23. Pharmazeutische Kombination zur oralen, parenteralen oder rektalen Verabreichung, umfassend:
(a) eine therapeutische wirksame Menge eines therapeutischen Wirkstoffs, der mindestens eine hämorrhagische Nebenwirkung verursacht, wobei der Wirkstoff ausgewählt ist aus der Gruppe, bestehend aus:
Dalteparin-Natrium, Sirudin, Xtransulfat, Enoxaparin-Natrium, Fluindion, Heparinat-Magnesium, Heparin-Calcium, Heparin-Natrium, Lepirudin, Nadroparin-Calcium, Pentosan-Polyestersulfat, Reviparin-Natrium, Tinzaparin-Natrium, Glycoaminoglycanen, Heparinen, unfraktioniertem Heparin, Standardheparin, niedermolekularen Heparinen, Heparinoiden, heparinartigen Molekülen und Gemischen davon und
(b) eine therapeutische wirksame Menge einer Verbindung der Formel (CH₃)₃N⁺(CH₂)ₙCOO⁻, worin n eine ganze Zahl von 1 bis 5 ist, eines pharmazeutisch annehmbaren Salzes davon oder von Gemischen davon zur Prävention oder Reduktion der hämorrhagischen Nebenwirkung und/oder zur Verstärkung der therapeutischen Wirkung des Wirkstoffes.

24. Pharmazeutische Kombination nach Anspruch 23, wobei die Verbindung der Formel (CH₃)₃N⁺(CH₂)ₙCOO⁻ Glycinbetain ist.

25. Pharmazeutische Kombination nach Anspruch 23, wobei Glycinbetain in einer Form, die zur subkutanen Injektion geeignet ist, oder in einer Form, die zur Herstellung einer Form zur subkutanen Injektion geeignet ist, vorliegt.

26. Pharmazeutische Kombination nach Anspruch 23, wobei der therapeutische Wirkstoff, der ein antithrombotisches Mittel mit Nebenwirkungen ist, und das Glycinbetain in einer Form vorliegen, die zur gleichzeitigen Verabreichung oder aufeinanderfolgenden Verabreichung oder zur Verabreichung auf verschiedenen Wegen geeignet ist.

## Revendications

1. Une combinaison pharmaceutique antithrombotique comprenant : (a) une quantité thérapeutique efficace d'un agent antithrombotique thérapeutiquement actif causant au moins un effet indésirable hémorragique, ledit agent actif étant choisi parmi le groupe constitué par :
des antiagrégants choisis parmi le groupe constitué de:
l'abciximab, l'acétylsalicylate d'aluminium basique, l'acétylsalicylate de carbonate de sodium, l'acétylsalicylate de lysine, l'acide acétylsalicylique, l'aloxiprine, le chlorydrate d'anagreli, le furamate de bencyclane, le carbasalate calcique, le clopidogrel sulfate, l'époprosténol sodique, l'epifibati, l'hydroxychloroquine sulfate, l'iloprost, la nicergoline, la nifepidine, le pyricarbate, la sulfinpyrazone, le chlorhydrate de ticlopidine, le chlorhydrate de tirofiban, le chlorhydrate de vérapamil, et leurs mélanges,
et/ou des anticoagulants choisis parmi le groupe constitué de:
l'acénocoumarol, l'anisindione, le biscoumacétate éthyle, la bromindione, le coumetarol, la sirudine, l'oxazidione, la phenindione, la phenprocoumone, tioclomarol, la warfarine sodique et leurs mélanges,
et/ou des fibrinolytiques choisis parmi le groupe constitué de:
l'altepase, l'anistreplase, l'atorvastatine calcique, les bromelaines, le ciprofibrate, le defibrotide, la fluvastatine sodique, le glicazide, la lovastatine, le lys-plasminogene, la phenformine, la pravastatine sodique, le reteplase, la simvastatine, la streptokinase, l'urokinase, et leurs mélanges ,
et/ou un inhibiteur de la thrombine choisi parmi le groupe constitué de l'argatroban, le novastan, et leurs mélanges,
et/ou les antivitamine K,
et leurs mélanges,
et (b) une quantité thérapeutique efficace d'un composé de formule (CH₃)₃N⁺(CH₂)ₙCOO⁻ où n est un entier de 1 à 5, ou un sel pharmaceutiquement acceptable de celui-ci, ou leurs mélanges, pour prévenir ou réduire ledit effet indésirable hémorragique et pour potentialiser l'activité thérapeutique antithrombotique dudit agent actif.

2. La combinaison pharmaceutique de la revendication 1, dans laquelle l'agent thérapeutique actif a au moins des effets indésirables hémorragiques possibles, et dans laquelle la combinaison comprend une quantité thérapeutique efficace de glycine bétaine afin de prévenir substantiellement ou complètement ledit effet indésirable hémorragique.

3. La combinaison pharmaceutique de la revendication 1, dans laquelle la glycine bétaine est sous une forme apte pour l'injection sous-cutanée ou dans une forme apte pour la préparation d'une forme pour l'injection sous-cutanée.

4. La combinaison pharmaceutique de la revendication 1, dans laquelle l'agent thérapeutique est choisi parmi le groupe constitué des antivitamine K, des antiagrégants, des anticoagulants, des anti thrombine, des fibrinolytiques et leurs mélanges.

5. La combinaison pharmaceutique de la revendication 1, dans laquelle l'agent thérapeutique actif qui est un agent antithrombotique et la glycine bétaine sont sous une forme apte pour l'administration simultanée ou pour l'administration successive ou pour l'administration selon différentes voies.

6. La combinaison pharmaceutique de la revendication 1, dans laquelle le composé de formule (CH₃)₃N⁺(CH₂)ₙCOO⁻ est la glycine bétaine.

7. Utilisation d'un composé de formule (CH₃)₃N⁺(CH₂)ₙCOO⁻ où n est un entier de 1 à 5, ou un sel pharmaceutiquement acceptable de celui-ci, leurs mélanges, comme un agent antidote actif pour la préparation d'une composition antidote pour prévenir thérapeutiquement des effets indésirables liés à un agent actif choisi parmi le groupe constitué par :
des antiagrégants choisis parmi le groupe constitué de :
l'abciximab, l'acétylsalicylate d'aluminium basique, l'acétylsalicylate de carbonate de sodium, l'acétylsalicylate de lysine, l'acide acétylsalicylique, l'aloxiprine, le chlorydrate d'anagreli, le furamate de bencyclane, le carbasalate calcique, le clopidogrel sulfate, l'époprosténol sodique, l'epifibati, l'hydroxychloroquine sulfate, l'iloprost, la nicergoline, la nifepidine, le pyricarbate, la sulfinpyrazone, le chlorhydrate de ticlopidine, le chlorhydrate de tirofiban, le chlorhydrate de vérapamil, et leurs mélanges,
et/ou des anticoagulants choisis parmi le groupe constitué de:
l'acénocoumarol, l'anisindione, le biscoumacétate éthyle, la bromindione, le coumetarol, la dalteparine sodique, la sirudine, le sulfate de xtran, l'enoxaparine sodique, la fluindione, l'héparinate magnésique, l'héparine calcique, l'héparine sodique, la lepirudine la nadroparine calcique, l'oxazidione, le pentosane polyester sulfurique, la phenindione, la phenprocoumone, la reviparine sodique, la tinzaparine sodique, le tioclomarol, la warfarine sodique, les glycoaminoglycanes, les héparines, les héparines non fractionnées, l'héparine standard, les héparines de bas poids moléculaires, les héparinoïdes, les molecules "héparine-like", et leurs mélanges,
et/ou des fibrinolytiques choisis parmi le groupe constitué de :
l'altepase, l'anistreplase, l'atorvastatine calcique, les bromelaines, le ciprofibrate, le defibrotide, la fluvastatine sodique, le glicazide, la lovastatine, le lys-plasminogene, la phenformine, la pravastatine sodique, le reteplase, la simvastatine, la streptokinase, l'urokinase, et leurs mélanges ,
et/ou un inhibiteur de la thrombine choisi parmi le groupe constitué de l'argatroban, le novastan, et leurs mélanges,
et/ou les antivitamine K,
et leurs mélanges.

8. L'utilisation de la revendication 7, dans laquelle le composé de formule (CH₃)₃N⁺(CH₂)ₙCOO⁻ est la glycine bétaine.

9. Utilisation de la glycine bétaine comme agent thérapeutique actif pour la préparation d'une composition pharmaceutique pour traiter ou prévenir un trouble causé par l'administration à un patient d'une quantité efficace d'un agent thérapeutique avec au moins un effet indésirable hémorragique, et/ou pour potentialiser l'effet thérapeutique dudit agent thérapeutique avec au moins un effet indésirable hémorragique.

10. Utilisation de la glycine bétaine pour la préparation d'une composition pharmaceutique injectable en sous-cutané pour traiter ou prévenir des troubles hémorragiques chez un patient, causés par l'administration au dit patient d'une quantité thérapeutique efficace d'un agent actif antithrombotique avec au moins un effet indésirable hémorragique.

11. Un système pharmaceutique de relargage contrôlé apte à délivrer après administration d'une manière contrôlée dans le temps dans la circulation sanguine d'un mammifère une glycine bétaine ou une quantité efficace d'un composé de formule (CH₃)₃N⁺(CH₂)ₙCOO⁻ avec n égal à 1 ou un sel pharmaceutiquement acceptable de ceux-ci ou leurs mélanges.

12. Le système de la revendication précédente qui est une préparation ou un dispositif oral de relargage contrôlé.

13. Un système pharmaceutique de relargage contrôlé selon la revendication 11, ledit système étant une préparation ou un dispositif transdermique de relargage contrôlé, spécialement un patch.

14. Le système de l'une quelconque des revendications 11 à 13 pour relarguer comme ingrédient actif la glycine bétaine.

15. Un dispositif pharmaceutique oral de relargage contrôlé selon la revendication 11 pour relarguer de manière contrôlée dans le temps pour au moins 120 minutes, après administration, une quantité thérapeutiquement efficace d'au moins une glycine bétaine ou une quantité efficace d'un composé de formule (CH₃)₃N⁺(CH₂)ₙCOO⁻ avec n égal à 1, leurs sels pharmaceutiquement acceptables ou leurs mélanges, pour traiter ou prévenir des désordres du flux sanguin.

16. Un dispositif pharmaceutique de relargage contrôlé selon la revendication 11 pour relarguer de manière contrôlée dans le temps pour au moins 120 minutes, après administration, une quantité thérapeutiquement efficace d'une glycine bétaine ou une quantité efficace d'un composé de formule (CH₃)₃N⁺(CH₂)ₙCOO⁻ avec n égal à 1, leurs sels pharmaceutiquement acceptables ou leurs mélanges, pour traiter ou prévenir la thrombose et/ou des désordres thromboemboliques.

17. Un dispositif pharmaceutique de relargage contrôlé selon la revendication 11 pour relarguer une quantité thérapeutique efficace d'au moins un composé choisi parmi le groupe constitué des bétaines, leurs sels pharmaceutiquement acceptables ou leurs mélanges, dans lequel le système contrôle pour au moins 120 minutes le relargage d'au moins une glycine bétaine ou d'une quantité efficace d'un composé de formule (CH₃)₃N⁺(CH₂)ₙCOO⁻ avec n égal à 1, leurs sels pharmaceutiquement acceptables ou leurs mélanges.

18. Le système de la revendication 17, dans lequel le système contrôle pour au moins 180 minutes le relargage d'au moins une glycine bétaine ou une quantité efficace d'un composé de formule (CH₃)₃N⁺(CH₂)ₙCOO⁻ avec n égal à 1, leurs sels pharmaceutiquement acceptables ou leurs mélanges.

19. Le système des revendications 17 ou 18, dans lequel le système contrôle pour au moins 240 minutes, avantageusement pour au moins 360 minutes, préférablement pour au moins 2160 minutes, le relargage d'au moins une glycine bétaine ou une quantité efficace d'un composé de formule (CH₃)₃N⁺(CH₂)ₙCOO⁻ avec n égal à 1, leurs sels pharmaceutiquement acceptables, leurs esters ou leurs mélanges.

20. Le système selon l'une quelconque des revendications 11 à 19, dans lequel le système comprend un ou plusieurs dispositif électronique ou puces contrôlant un ou plusieurs système ou dispositif de relargage.

21. Utilisation de glycine bétaine comme agent actif pour la préparation d'un système pharmaceutique de relargage contrôlé pour traiter ou prévenir un trouble du flux sanguin et/ou la thrombose et/ou des désordres thromboemboliques.

22. Utilisation selon la revendication 21 pour l'administration transdermique de la glycine bétaine pour traiter ou prévenir un trouble du flux sanguin et/ou la thrombose et/ou des désordres thromboemboliques.

23. Une combinaison pharmaceutique pour l'administration orale, parentérale ou rectale comprenant :
(a) une quantité thérapeutique efficace d'un agent thérapeutiquement actif causant au moins un effet indésirable hémorragique, le dit agent actif étant choisi parmi le groupe constitué par :
la dalteparine sodique, la sirudine, le sulfate de xtran, l'enoxaparine sodique, la fluindione, l'héparinate magnésique, l'héparine calcique, l'héparine sodique, la lepirudine nadroparine calcique, le pentosane polyester sulfurique, la reviparine sodique, la tinzaparine sodique, les glycoaminoglycanes, les héparines, l'héparine non fractionnée, l'héparine standard, les héparines de bas poids moléculaires, les héparinoïdes, les molécules héparine-like, et leurs mélanges et
(b) une quantité thérapeutique efficace d'un composé de formule (CH₃)₃N⁺(CH₂)ₙCOO⁻ où n est un entier de 1 à 5, un sel pharmaceutiquement acceptable de celui-ci, ou leurs mélanges, pour prévenir ou réduire ledit effet indésirable hémorragique et/ou pour potentialiser l'effet thérapeutique antithrombotique dudit agent actif.

24. La combinaison pharmaceutique de la revendication 23, dans laquelle le composé de formule (CH₃)₃N⁺(CH₂)ₙCOO⁻ est la glycine bétaine.

25. La combinaison pharmaceutique de la revendication 23, dans laquelle la glycine bétaine se trouve sous une forme apte pour l'injection sous-cutanée ou sous une forme apte pour la préparation d'une forme pour l'injection sous cutanée.

26. La combinaison pharmaceutique de la revendication 23, dans laquelle l'agent thérapeutique actif qui est un agent antithrombotique avec des effets indésirables et la glycine bétaine sont sous une forme apte pour l'administration simultanée ou l'administration successive ou pour l'administration selon des voies différentes.
